Europäisches Patentamt
European Patent Office
Office européen des brevets

(19)

(11) Publication number: **0 007 672 A1**

(12) # EUROPEAN PATENT APPLICATION


(21) Application number: 79200404.6

(22) Date of filing: 16.07.79

(51) Int. Cl.³: C 07 C 147/08
C 07 D 307/52, A 61 K 31/275
A 61 K 31/34, A 01 N 47/40

(30) Priority: 17.07.78 NL 7807631

(43) Date of publication of application: 06.02.80 Bulletin 80/3

(84) Designated Contracting States: AT BE CH DE FR GB IT LU NL SE



(71) Applicant: Gist-Brocades N.V.
Wateringseweg 1 P.O. Box 1
NL-2600 MA Delft(NL)

(72) Inventor: Van Leusen, Albert Mattheus
Savorin Lohmannlaan 23
NL-9722 HC Groningen(NL)

(72) Inventor: Akkerboom, Piet Johannes
Dr. J.W.Palthelaan 48
NL-2712 RT Zoetermeer(NL)

(74) Representative: Kok, Pieter Nicolaas et al,
c/o Gist-Brocades N.v. Patents and Trade Marks
Wateringseweg 1 P.O. Box 1
NL-2600 MA Delft(NL)


(54) **Isocyanoalkenes, process and useful intermediates for their preparation, pharmaceutical and fungicidal preparations containing those isocyanoalkenes and the use of the fungicidal preparations in agriculture.**


(57) Isocyanoalkenes, process and useful intermediates for their preparation, pharmaceutical and fungicidal preparations containing those isocyanoalkenes and the use of the fungicidal preparations in agriculture, the isocyanoalkenes having the general formula:

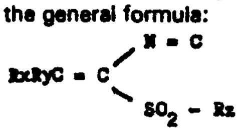

or

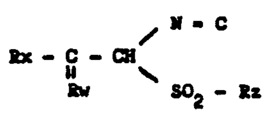

wherein Rx and Ry are the same or different and each is a hydrogen atom or an alkyl, aryl or heterocyclic group and Rx and Ry together with the carbon atom to which they are attached represent a homo- or heterogeneous cycloalkylidene, adamantylidene or cholestanylidene group, Rw is an alkylidene group or Rw together with Rx and the carbon atom to which they are attached represent a homo- or heterogeneous cycloalkenyl group and Rz is an aryl group.


EP 0 007 672 A1


Croydon Printing Company Ltd.

Isocyanoalkenes, process and useful intermediates for their preparation, pharmaceutical and fungicidal preparations containing those isocyanoalkenes and the use of the fungicidal preparations in agriculture.

The invention relates to novel 1-isocyano-1-sulphonylalkenyl derivatives showing antimycotic, antibacterial and antitrichomonas properties.

According to the invention there are provided novel isocyanoalkenes having the general formula:

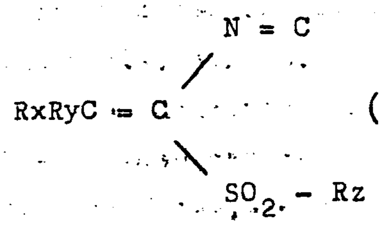 (I) or 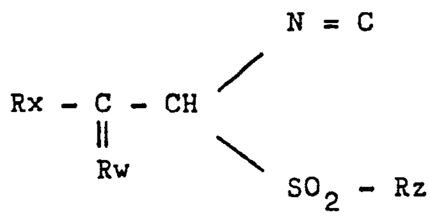 (II)

wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five- or six-membered heterocyclic group bearing one or more oxygen, sulphur or nitrogen atoms as hetero atoms, substituted or not substituted by one or more lower alkyl, aryl or halogen groups or Rx and Ry, together with the carbon atom to which they are attached represent a five- to eight-membered cycloalkylidene group which may bear one or more oxygen, sulphur or nitrogen atoms as hetero atoms, or an adamantylidene or cholestanylidene group, substituted or not substituted with one or more lower alkyl groups, Rw is a lower alkylidene group or Rx and Rw, together with the carbon atom to which they are attached represent a five- to eight-membered cycloalkenyl group which may bear one or more oxygen, sulphur or nitrogen atoms as hetero atoms, substituted or not substituted by one or more lower alkyl groups, and Rz is an aryl group substituted or not substituted by one or more lower alkyl groups.

Although the compounds according to formula (I) have been mentioned in a general way in O. Possel's thesis, the title of which is "Synthese en Reakties van Gesubstitueerde Tosylmethylisocyaniden" (Synthesis and Reactions of Substituted p-Toluenesulphonylmethylisocyanides) which was upheld on 19th June 1978, that thesis does not show more than the general formula and the remark that those compounds may be obtained by dehydration of the corresponding formamides. Therefore the advantageous properties of the compounds according to the invention are not evident from this thesis.

Preferred isocyanoalkenes are those having formula (I), wherein Rx, Ry and Rz have the above-indicated significance, Rx more preferably being an aryl or five-membered heteroaryl group which may bear an oxygen, sulphur or nitrogen atom as the hetero-atom, and optionally being substituted by a lower alkyl, nitro, lower alkoxy, or halogen group, Ry preferably being a hydrogen atom or a lower alkyl group and Rz having the above-indicated significance.

Compounds representing the invention are 1-isocyano-2-phenyl-1-(4-toluenesulphonyl)ethene, α-adamantylidene-α-(4-toluene-sulphonyl)methylisocyanide, 1-isocyano-2-(4-nitrophenyl)-1-(4-toluenesulphonyl)ethene, 3,3-dimethyl-1-isocyano-1-(4-toluenesulphonyl)-1-butene, 1-isocyano-2-methyl-1-(4-toluenesulphonyl)-1-propene, 2,2-diphenyl-1-isocyano-1-(4-toluenesulphonyl)-ethene, α-cyclohexyl-idene-α-(4-toluenesulphonyl)-methylisocyanide, 1-isocyano-1-(4-toluenesulphonyl)-ethene, α-cyclopentylidene-α-(4-toluenesulphonyl)-methylisocyanide, 2-(2-furanyl)-1-isocyano-1-(4-toluenesulphonyl)-ethene, 1-isocyano-2-(4-chlorophenyl)-1-(4-toluenesulphonyl)-ethene, 2-phenyl-1-isocyano-1-(4-toluenesulphonyl)-1-propene, 1-isocyano-2-(2-furanyl)1-(4-toluenesulphonyl)-1-propene, α-(tetrahydropyran-4-ylidene)-α-(4-toluenesulphonyl)-methylisocyanide, α-(2,3-dihydro-6H-pyran-4-yl)-α-(toluenesulphonyl)-methylisocyanide, 1-isocyano-1-(4-toluenesulphonyl)-1-nonene, α-(thian-4-ylidene)-α-(4-toluene-sulphonyl)-methylisocyanide, α-(3,4-dehydrothian-4-yl)-α-(4-toluenesulphonyl)-methylisocyanide, 1-isocyano-1-(4-toluenesulphonyl)-2-(2-thienyl)-1-propene, α-cycloheptylidene-α-(4-toluenesulphonyl)-methyl-isocyanide, α-(cyclohepten-1-yl)-α-(4-toluenesulphonyl)-methylisocyanide, 2-phenyl-1-isocyano-1-(4-toluenesulphonyl)-1-hexene, α-(cycloocten-1-yl)-α-(4-toluenesulphonyl)-methylisocyanide, 1-isocyano-2-methyl-1-(4-toluenesulphonyl)-undecene, 1-isocyano-2-(4-nitrophenyl)-1-(4-toluenesulphonyl)-1-propene, 1-isocyano-2-(4-nitrophenyl)-1-(4-toluenesulphonyl)-2-propene, 1-isocyano-2-(4-methoxyphenyl)-1-(4-toluenesulphonyl)-ethene, 1-isocyano-1-(4-toluenesulphonyl)-1-pentene, α-cyclohexylidene-α-benzenesulphonyl-methyl-isocyanide, 1-isocyano-2-pentyl-1-(4-toluenesulphonyl)-1-heptene, 1-isocyano-2-pentyl-1-(4-toluenesulphonyl)-1-heptene, 1-isocyano-2-methyl-1-(4-toluenesulphonyl)-1-butene, 1-isocyano-2-methyl-1-(4-toluenesulphonyl)2-butene, α-(3-cholestanylidene)-α-(4-toluenesulphonyl)-methylisocyanide, 1-isocyano-2-methyl-1-(4-toluenesulphonyl)-4-(1,1,3-trimethylcyclohex-2-en-2-yl)-3-trans-1,3-butadine, 2-[1-isocyano-1-(4-toluenesulphonyl)-ethen-2-yl]-1-methylpyrrole and 2-(4-phenylphenyl)-1-isocyano-1-(4-toluenesulphonyl)-1-propene.

By the term "lower" as used in connection with alkyl and alkoxy groups, those groups are meant that possess 1 to 6 carbon atoms. Those alkyl or alkoxy groups in question may possess one or more unsaturated bonds.

By the term "aryl" are meant phenyl, naphthyl, phenanthryl or anthracyl groups, preferably phenyl groups. By the term "five-membered aryl group" is meant a five-membered cyclic structure having two double bonds.

The compounds according to the invention are active against infections, and they possess in particular antimycotic properties so that they may be used in human and veterinary medical science. In addition they may be used for combatting plant diseases, in particular as fungicides. A list of MIC (Minimum Inhibitory Concentration) values for a number of microorganisms is listed herebelow. The figures in the column headings refer to the compounds prepared according to the experimental examples in comparison with the spectrum of 1-[2-(2,4-dichlorophenyl)-2-{(2,4-dichlorophenyl)-methoxy}-ethyl]-1H-imidazole (better known under the name of miconazole, c.f. Merck Index 9th Ed. page 6047) or natamycin (pimaricin, c.f. Merck Index 9th Ed. page 7233).

| | MIC values in µg/ml | | | | | nata- |
|---|---|---|---|---|---|---|
| Examples: | 1 | 7 | 9 | 10 | 13 | mycin |
| Aspergillus flavus A 2385 | | | | | 12.5 | 50 |
| " versiodor Nr. 7 | | | | | >100 | 6 |
| " parasiticus A 2396 | | | | | 6 | 2.5 |
| " " A 2397 | | | | | 6 | 2.5 |
| Fusarium A 3241 | | | | | 50 | 3 |
| " solani A 3245 | 50 | 12.5 | 12.5 | 25 | | 9.3 |
| " (bulb) A 3252 | >100 | 18.8 | 25 | 25 | | 6 |
| " (tulip) A 3253 | 50 | 12.5 | 12.5 | 12.5 | | 6 |
| Penicillium islandicum D 189 | | | | | 12.5 | 3 |
| " italicum 278-65 | | | | | 3 | 3 |
| " patalum A 2389 | | | | | 25 | 3 |
| " rubrum A 2395 | | | | | 25 | 3 |
| " veridicatum A 2370 | | | | | 3 | 1.5 |
| Rhizoctonia solani 200-25 | 4.5 | 1.5 | 1.5 | 1.5 | 6 | 2.3 |

MIC values in µg/ml

| Example: | 1 | 2 | 4 | 5 | 7 | 9 | 10 | 11 | 12 | 13 |
|---|---|---|---|---|---|---|---|---|---|---|
| Candida albicans A 3140 | 12.5 | 12.5 | 12.5 | 12.5 | 3 | 1.5 | 0.2 | 0.4 | 6 | 0.2 |
| " " A 3141 | 12.5 | >100 | >100 | 25 | 12.5 | 3 | 0.4 | 0.8 | 6 | <0.05 |
| " " A 3142 | 12.5 | >100 | 50 | 25 | 12.5 | 1.5 | 0.4 | 1.5 | 6 | |
| " " A 3143 | 12.5 | 0.8 | 6 | 25 | 6 | 0.8 | 0.2 | 1.5 | 6 | |
| " " A 3144 | 12.5 | >100 | 50 | 25 | 6 | 1.5 | 0.2 | 1.5 | 3 | 0.2 |
| " " A 3145 | 12.5 | >100 | >100 | 12.5 | 6 | 1.5 | 0.8 | 1.5 | 3 | <0.05 |
| " " A 3165 | 25 | 50 | 50 | 12.5 | 6 | 3 | 0.4 | 1.5 | 3 | 1.5 |
| " " A 3166 | 12.5 | 50 | 12.5 | 12.5 | 6 | 6 | 0.4 | 1.5 | 3 | |
| " " A 3167 | 25 | 25 | 50 | 25 | 6 | 3 | 0.4 | 1.5 | 3 | 0.4 |
| " " A 3168 | 12.5 | | 50 | 25 | 6 | 6 | 0.4 | 0.8 | 6 | 0.2 |
| " " A 3169 | 12.5 | 1.5 | 100 | 25 | 6 | 3 | 0.4 | 1.5 | 6 | 0.2 |
| " " A 3171 | 12.5 | >100 | 50 | 12.5 | 6 | 3 | 0.4 | 1.5 | 6 | <0.05 |
| " " A 3184 | 12.5 | 0.8 | 100 | 12.5 | 3 | 1.5 | 0.2 | 1.5 | 6 | |
| " " A 3190 | 6 | 100 | 50 | 12.5 | 3 | 1.5 | 0.4 | 1.5 | 6 | <0.05 |
| " " A 3191 | 12.5 | | 50 | 25 | 6 | 0.8 | 0.4 | 1.5 | 6 | <0.05 |
| " " A 3192 | 6 | 1.5 | 25 | 25 | 6 | 0.8 | 0.2 | 1.5 | 3 | |
| " " A 3194 | 25 | >100 | >100 | 25 | 6 | 0.8 | 0.4 | 1.5 | 3 | <0.05 |
| " " A 3196 | 12.5 | >100 | 25 | 25 | 12.5 | 3 | 0.4 | 3 | 6 | <0.05 |
| " " A 7 | 1.5 | 0.8 | 6 | 3 | 0.8 | 0.8 | 0.2 | 0.8 | 3 | 0.1 |
| Pasteurella multocida A 723 | | >100 | 3 | | | 3 | <1.5 | 6 | 3 | 0.75 |
| Streptococcus haemolyticus A 1088 | | 50 | 1.5 | | | 25 | 6 | 3 | 50 | |
| Bacillus subtilis ATCC 6633 | | | 12.5 | | | | 6 | | | 1,5 |
| Staphylococcus aureus A 321 | | | 12.5 | | | | 12.5 | | | 12.5 |
| " " A 355 | | | 6 | | | | 12.5 | | | 6 |
| " " A 2000 | | | 6 | | | | 12.5 | | | 6 |
| Diplococcus pneumoniae A 2783 | | | 6 | | | | 6 | | | 12.5 |
| Proteus morganii 2241 | | | 100 | | | | 6 | | | 12.5 |

MIC values in µg/ml

| Example: | 1 | 2 | 4 | 5 | 7 | 9 | 10 | 11 | 12 | 13 |
|---|---|---|---|---|---|---|---|---|---|---|
| Trichophyton equinum 182-76 | 3 | 50 | 3 | 3 | 3 | 3 | 1,5 | 3 | 12.5 | 1.5 |
| " " 441-51 | | 25 | <0.05 | | | 0.1 | <0.05 | 0.4 | | |
| " " 856-71 | 3 | >100 | 0.2 | 6 | 3 | <0.05 | <0.05 | <0.05 | 6 | 0.1 |
| " mentagrophytus A 3252 | 1.5 | >100 | 1.5 | 0.8 | 0.8 | 1.5 | 3 | 1.5 | 6 | 3 |
| " " R 177 | 6 | 100 | 6 | 6 | 3 | 3 | 1.5 | 3 | 3 | 3 |
| " " R 178 | >100 | >100 | >100 | 50 | 12.5 | 12.5 | 100 | >100 | 6 | 12.5 |
| " " RRL 305 | 6 | 50 | 3 | 3 | 1.5 | 0.8 | 0.1 | 1.5 | 25 | 3 |
| " " 367-68 | 1.5 | >100 | 0.2 | 1.5 | 0.8 | 3 | 0.1 | 1.5 | 12.5 | 3 |
| " " 421-67 | 1.5 | 50 | 0.8 | 1.5 | 1.5 | 6 | 1.5 | 6 | 6 | 3 |
| " quickeaneum A 2040 | 3 | >100 | 3 | 0.8 | 0.8 | 1.5 | 3 | 1.5 | 6 | 0.4 |
| " rubrum A 810 | | 50 | 6 | | 0.8 | 0.1 | <0.05 | 0.8 | 3 | 0.8 |
| " " 218-65 | | <0.05 | 0.1 | 0.4 | | 0.8 | 0.1 | 1.5 | 3 | 0.8 |
| " " 494-62 | 0.1 | >100 | 0.8 | 0.4 | 0.8 | 1.5 | 0.1 | 1.5 | 6 | 0.8 |
| " schoenleinii 435-63 | 0.1 | 25 | <0.05 | 0.2 | 0.2 | 0.4 | 0.1 | 0.4 | 1.5 | 3 |
| " soudanese joyeux 440-63 | 12.5 | 50 | 3 | 12.5 | 12.5 | 6 | 6 | 6 | | 6 |
| " tonsurans 561-50 | | | 1.5 | <0.1 | | 6 | 3 | 6 | 0.1 | 3 |
| " verrucosum A 2922 | | <0.05 | | | | 0.4 | <0.05 | 0.4 | | 3 |
| " " N 3 | | | 0.1 | | | | | | | |
| " " 134-66 | | 25 | <0.05 | | | <0.05 | 0.2 | <0.05 | | 0.8 |
| " violoceum 442-62 | 0.1 | 50 | 0.05 | 0.1 | 0.2 | 0.4 | 0.1 | 0.1 | 3 | 3 |

MIC values in µg/ml

| Example: | 14a | 14b | 16a | 16b | 17 | 18a | 18b | 19 | 20 | 22 |
|---|---|---|---|---|---|---|---|---|---|---|
| Candida albicans A 3140 | 12.5 | 6 | 6 | 3 | 1.5 | 1.5 | 6 | 6 | 6 | 3 |
| " " A 3141 | 12.5 | 12.5 | 6 | 3 | 1.5 | 3 | 12.5 | 6 | 6 | 3 |
| " " A 3142 | 12.5 | 6 | 6 | 3 | 1.5 | 1.5 | 12.5 | 6 | 6 | 3 |
| " " A 3143 | 12.5 | 6 | 12.5 | 3 | 1.5 | 1.5 | 12.5 | 6 | 6 | 1.5 |
| " " A 3144 | 25 | 25 | 12.5 | 6 | 1.5 | 3 | 12.5 | >100 | 6 | 3 |
| " " A 3145 | 12.5 | 12.5 | 12.5 | 6 | 3 | 3 | 6 | 6 | 6 | 3 |
| " " A 3165 | 12.5 | 6 | 6 | 3 | 1.5 | 1.5 | 12.5 | 6 | 6 | 3 |
| " " A 3166 | 6 | 6 | 3 | 3 | 1.5 | 3 | 12.5 | 6 | 6 | 1.5 |
| " " A 3167 | 6 | 6 | 3 | 3 | 1.5 | 0.75 | 12.5 | 50 | 6 | 1.5 |
| " " A 3168 | 6 | 6 | 3 | 3 | 3 | 0.75 | 6 | 6 | 6 | 3 |
| " " A 3169 | 6 | 6 | 12.5 | 3 | 1.5 | 3 | 12.5 | 3 | 6 | 3 |
| " " A 3171 | 6 | 6 | 6 | 3 | 1.5 | 1.5 | 12.5 | 3 | 6 | 3 |
| " " A 3184 | 12.5 | 6 | 12.5 | 3 | 1.5 | 3 | 12.5 | 50 | 6 | 1.5 |
| " " A 3190 | 12.5 | 12.5 | 25 | 3 | 1.5 | 3 | 12.5 | 6 | 6 | 3 |
| " " A 3191 | 12.5 | 25 | 25 | 6 | 3 | 3 | 12.5 | >100 | 6 | 3 |
| " " A 3192 | 12.5 | 6 | 25 | 3 | 1.5 | 1.5 | 12.5 | 6 | 6 | 3 |
| " " A 3194 | 12.5 | 12.5 | 25 | 3 | 3 | 1.5 | 25 | 6 | 3 | 1.5 |
| " " A 3196 | 12.5 | 25 | 25 | 3 | 1.5 | 3 | 12.5 | 100 | 6 | 3 |
| " " A 7 | 12.5 | 6 | 6 | 3 | 0.75 | 1.5 | 6 | 6 | 6 | 1.5 |
| Pasteurella multocida A 723 | 6 | 6 | 6 | 3 | 3 | 20 | 50 | >100 | 50 | 6 |
| Streptococcus haemolyticus A 1088 | 25 | 100 | 25 | 50 | 6 | 10 | 25 | >100 | 12.5 | 0.8 |

MIC values in µg/ml

| Example: | 14a | 14b | 16a | 16b | 17 | 18a | 18b | 19 | 20 | 22 |
|---|---|---|---|---|---|---|---|---|---|---|
| Trichophyton equinum 182-76 | 25 | 25 | 25 | 6 | >100 | | >100 | >100 | 50 | |
| " " 441-51 | 6 | 3 | 3 | | 0.75 | | <0.05 | 3 | 12.5 | 1.5 |
| " " 856-71 | | | | 12.5 | | | 100 | | | 25 |
| " mentagrophytus A 3252 | 25 | 25 | 25 | 12.5 | 6 | | >100 | >100 | 25 | 12.5 |
| " " R 177 | 12.5 | 25 | 12.5 | 6 | >100 | | >100 | >100 | 50 | 6 |
| " " R 178 | 100 | 50 | 100 | 12.5 | >100 | | >100 | >100 | >100 | 6 |
| " " RRL 305 | | | | 12.5 | | | >100 | | | 6 |
| " " 367-68 | 25 | 25 | 25 | 6 | 100 | | 25 | >100 | 50 | 3 |
| " " 421-67 | 25 | 25 | 50 | 6 | >100 | | >100 | >100 | 100 | 12.5 |
| " quickeaneum A 2040 | 25 | 25 | 12.5 | 6 | 6 | | 25 | >100 | 25 | |
| " rubrum A 810 | 12.5 | 12.5 | 3 | 1.5 | 12.5 | | | >100 | 100 | 3 |
| " " 218-65 | 3 | 3 | 6 | 1.5 | 6 | | 100 | >100 | 12.5 | 3 |
| " " 494-62 | 25 | 25 | 25 | 12.5 | 6 | | >100 | >100 | 12.5 | 6 |
| " schoenleinii 435-63 | 6 | 6 | 6 | 1.5 | 3 | | 6 | >100 | 12.5 | 3 |
| " soudanese joyeux 440-63 | | | | | | | | | | 3 |
| " tonsurans 561-50 | 0.75 | 0.4 | 0.4 | 0.8 | <0.4 | | | 0.4 | 0.75 | 1.5 |
| " verrucosum A 2922 | 3 | 1.5 | 1.5 | | 0.4 | | 12.5 | 3 | 6 | |
| " " N 3 | | | | | | | | | | |
| " " 134-66 | 3 | 3 | 3 | | 0.75 | | 3 | 3 | 6 | 1.5 |
| " violoceum 442-62 | 12.5 | 12.5 | 6 | 3 | 3 | | 6 | >100 | 12.5 | 3 |

MIC values in μg/ml

| Example: | 23 | 24 | 25 | 26 | 27a* | 27b | 30 | + | Δ | |
|---|---|---|---|---|---|---|---|---|---|---|
| Candida albicans A 3140 | 1.5 | 25 | 1.5 | 6 | 0.8 | 0.8 | 12.5 | 25 | 0.1 | |
| " " A 3141 | 1.5 | 25 | 1.5 | 25 | <0.05 | <0.05 | 12.5 | 3 | <0.05 | |
| " " A 3142 | 1.5 | 3 | 1.5 | 25 | | | 12.5 | | | |
| " " A 3143 | 1.5 | 12.5 | 1.5 | 50 | | | 6 | | | |
| " " A 3144 | 1.5 | 12.5 | 3 | 100 | 0.2 | 0.4 | 6 | 3 | 0.8 | |
| " " A 3145 | 1.5 | 12.5 | 3 | 25 | <0.05 | <0.05 | 12.5 | 3 | <0.05 | |
| " " A 3165 | 1.5 | 25 | 3 | 50 | 0.8 | 1.5 | 6 | 25 | 0.2 | |
| " " A 3166 | 1.5 | 50 | 1.5 | 25 | 3 | 3 | 6 | 6 | | |
| " " A 3167 | 1.5 | 50 | 3 | >100 | 0.2 | 0.4 | 3 | 1.5 | <0.05 | |
| " " A 3168 | 1.5 | 50 | 6 | 100 | 0.2 | 0.4 | 12.5 | 12.5 | 100 | |
| " " A 3169 | 1.5 | 25 | 1.5 | 25 | 0.1 | 0.4 | 6 | 1.5 | 1.5 | |
| " " A 3171 | 1.5 | 25 | 3 | 50 | 0.2 | 0.4 | 12.5 | 6 | <0.05 | |
| " " A 3184 | 1.5 | 25 | 1.5 | 50 | | | 6 | | | |
| " " A 3190 | 1.5 | 25 | 3 | 50 | <0.05 | 0.1 | 12.5 | | <0.05 | |
| " " A 3191 | 1.5 | 25 | 6 | >100 | 0.1 | 0.2 | >100 | 6 | <0.05 | |
| " " A 3192 | 1.5 | 25 | 1.5 | 50 | | | 6 | | | |
| " " A 3194 | 1.5 | 25 | 3 | 50 | <0.05 | 0.4 | 12.5 | 3 | 0.2 | |
| " " A 3196 | 1.5 | 25 | 3 | 50 | 0.4 | 0.4 | 12.5 | 6 | | |
| " " A 7 | 1.5 | 25 | 1.5 | 50 | 0.2 | 0.4 | 6 | 12.5 | 0.2 | |
| Pasteurella multocida A 723 | 3 | 50 | 3 | 100 | 1.5 | 3 | 25 | | >100 | |
| Streptococcus haemolyticus A 1088 | <0.05 | 100 | 25 | >100 | | | 25 | | | |
| Bacillus subtilis ATCC 6633 | 3 | | | | | | | | | |
| Staphylococcus aureus A 321 | 3 | | | | | | | | | |
| " " A 355 | 3 | | | | | | | | | |
| " " A 2000 | 1.5 | | | | | | | | | |
| Diplococcus pneumoniae A 2783 | 6 | | | | | | | | | |
| Proteus morganii 2241 | 6 | | | | | | | | | |

* 1:1 mixture of E and Z isomers
+ natamycin
Δ miconazole

MIC values in µg/ml

| Example: | 23 | 24 | 25 | 26 | 27a* | 27b | 30 | + | Δ | |
|---|---|---|---|---|---|---|---|---|---|---|
| Trichophyton equinum 182-76 | | 50 | 6 | >100 | 0.4 | 0.8 | | 25 | 0.4 | |
| " " 441-51 | 1.5 | | | | | | 0.4 | | | |
| " " 856-71 | 100 | 50 | 12.5 | 50 | <0.05 | 0.4 | >100 | 6 | <0.05 | |
| " mentagrophytus A 3252 | 100 | 25 | 3 | >100 | 6 | 3 | >100 | 100 | 0.4 | |
| " " R 177 | 12.5 | 25 | 3 | >100 | 6 | 6 | 100 | 50 | 0.4 | |
| " " R 178 | >100 | 25 | 3 | >100 | 6 | 6 | >100 | 50 | 25 | |
| " " RRL 305 | 25 | 50 | 3 | >100 | 3 | 3 | >100 | 12.5 | 0.4 | |
| " " 367-68 | 25 | 100 | 1.5 | >100 | 6 | 3 | 50 | 12.5 | 3 | |
| " " 421-67 | 100 | 50 | 1.5 | 100 | 6 | 3 | >100 | 12.5 | 0.2 | |
| " quickeaneum A 2040 | | 100 | 3 | >100 | 0.1 | 1.5 | | 12.5 | 0.1 | |
| " rubrum A 810 | 25 | 25 | 0.4 | 50 | 0.4 | 0.8 | 50 | 12.5 | 0.8 | |
| " " 218-65 | 25 | 25 | 0.4 | 100 | 0.4 | 1.5 | 50 | 12.5 | 0.1 | |
| " " 494-62 | 100 | 50 | 6 | 100 | 0.4 | 0.8 | >100 | 25 | 0.8 | |
| " schoenleinii 435-63 | 3 | 25 | 0.8 | 100 | 6 | 6 | 25 | 25 | 0.2 | |
| " soudanese joyeux 440-63 | 6 | | | | 6 | 6 | 6 | 25 | 1.5 | |
| " tonsurans 561-50 | 0.8 | 25 | 0.2 | 100 | 3 | 3 | 0.4 | 12.5 | 0.1 | |
| " verrucosum A 2922 | | | | | 6 | 6 | | 25 | 0.8 | |
| " " N 3 | | | | | | | | | | |
| " " 134-66 | 1.5 | | | | 0.8 | 1.5 | 6 | 25 | 0.8 | |
| " violoceum 442-62 | 3 | 25 | 3 | 100 | 12.5 | 6 | 6 | 50 | 0.8 | |

* 1:1 mixture of E and Z isomers
\+ natamycin
Δ miconazole

The table shows that the compounds according to the invention generally possess a good activity against Trichophyton, Candida albicans and some plant moulds.

Additionally the compounds according to the invention are valuable intermediates for the preparation, in high yields, of imidazoles that may be substituted on the 1st and/or 5th ring carbon atom. These imidazoles are prepared by reacting the 1-isocyano-1-sulphonylethenyl derivatives with a primary amine or with ammonia, under removal of the $SO_2Rz$ group. The imidazoles may be useful, inter alia, for stabilisation of meat colour, as a catalyst in epoxy resin reactions, as accelerators in photographic developers, and as stabiliser in enzyme preparations for cleaning purposes.

The compounds according to the invention may be prepared by treating a compound, having the general formula:

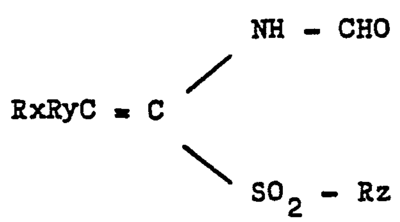

(III)

wherein Rx, Ry and Rz are as indicated above, with a dehydrating agent. Any method for the preparation of an isocyanide from a formamide may be used, for example the reaction with phosphorusoxychloride. That reaction is preferably carried out at lower temperatures, e.g. between -50 and +25°C, preferably between -30 and -5°C. Other dehydrating agents may, however, also be used. Examples thereof are phosgene, thionyl chloride, cyanuryl chloride, alkyl- and arylsulphonyl chloride, a mixture of triphenylphosphine, carbon tetrachloride and triethyl amine, 2-chloro-3-ethylbenzoxazolium tetrafluoroborate or phosphorus tri- or pentachloride (c.f. Ugi, Isonitril Chemistry, 1971, Acad. Press New York, pages 10 to 16 for a more complete review) and diphosgene (c.f. Skorna and Ugi, Angew. Chemie 89 (1977) page 267). The dehydration is preferably carried out in the presence of an acid-binding agent, such as an amine. Examples of suitable amines are triethyl amine, substituted or unsubstituted pyridines, N-methylmorpholine, while other alkaline agents may be used too, such as potassium carbonate, sodium carbonate, potassium t-butylate and in special cases even sodium hydroxide. The dehydration is preferably carried out in an inert organic solvent, such as di-, tri- or tetrachloromethane, ethyl

acetate, dioxan, benzene, toluene, xylene, o-dichlorobenzene, acetone, 1,2-dimethoxyethane, bis-(2-methoxyethyl)-ether, dimethylformamide or 1,2-dichloroethane or mixtures thereof. Generally the compounds of formula (I) are formed, but sometimes mixtures of compounds of formula (I) and those of formula (II) are formed, or even compounds of formula (II) alone. It depends more or less on the pKa of the amine, which of the compounds are formed in favour.

The compounds of formula (II) may be obtained by treating the corresponding sulphomethylisocyanide with the corresponding aldehyde or ketone and a strong alkaline agent as indicated by Schöllkopf et al, Angew. Chemie, Int. Ed. (Engl.) 12 No. 5 (1973) pages 407 to 408. Examples of useful strong alkaline agents are alkali metal t-butylate, alkali metal ethanolate, alkali metal hydride, butylated alkali metals, in which the alkali metal is generally lithium, sodium or potassium. Potassium t-butylate is preferably used. The reaction with the alkaline compound may be carried out at room temperature, but it is preferably carried out at lower temperatures, e.g. between -80 and +45°C, preferably between -60 and -30°C, dependent on the solvent used. The reaction is preferably carried out in an inert gas atmosphere, e.g. a nitrogen gas atmosphere. Higher yields are generally obtained in this way. The reaction is further preferably carried out in a polar organic solvent such as tetrahydrofuran, dimethylformamide, 1,2-dimethoxyethane, dimethylsulphoxide, hexamethylphosphorustriamide, dioxane, toluene or mixtures thereof.

It is an advantage of the invention that the preparation of the compounds of formula (III) and those of formula (I) or (II) may be combined to a "one pot reaction" in many cases.

Therefore it is also a feature of the invention to provide a process for the preparation of compounds of formula (I) or (II) in which a compound having the formula:

$$Rz - SO_2 - CH_2 - N = C \qquad (IV)$$

wherein Rz is as indicated above, is treated with a strong alkaline agent and a compound having the formula Rx - CO - Ry, wherein Rx and Ry are as indicated above, at lower temperatures and in an inert gas atmosphere, characterized in that the compound is treated with a dehydrating agent in a "one pot process".

It is appreciated to remark that a number of the intermediates of formula (III) are new and thus form a feature of the invention. In general the following compounds, which also form a feature of the invention, are new:
formamides having the general formula:

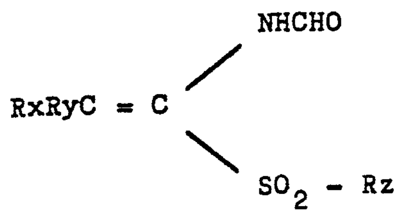

(V)

wherein Rx and Ry are the same or different and each is a hydrogen atom or an alkyl group having 1 to 10 carbon atoms or an aryl group, substituted or not substituted by one or more nitro, halogen, lower alkyl, cycloalkyl,lower alkoxy or aryl groups, or a five- or six-membered heterocyclic group bearing one or more oxygen, sulphur or nitrogen atoms as hetero atoms, substituted or not substituted by one or more lower alkyl, aryl or halogen groups, with the provision that, if Ry is a hydrogen atom, Rx is not a phenyl or t-butyl group, or Rx and Ry, together with the carbon atom to which they are attached, represent a five- to eight-membered cycloalkylidene group which may bear one or more oxygen, sulphur or nitrogen atoms as hetero atoms, with the exception of a cyclohexylidene group, or represent an adamantylidene or cholestanylidene group, substituted or not substituted by one or more lower alkyl groups, and Rz is an aryl group substituted or not substituted by one or more lower alkyl groups.

Formamides that are preferred according to the invention are those wherein Rx is an aryl or five-membered heteroaryl group, which may bear an oxygen, sulphur or nitrogen atom as the heterocyclic atom, and may be substituted by a lower alkyl, nitro, lower alkoxy or halogen group, Ry is a hydrogen atom or a lower alkyl group, with the provision that, if Ry is a hydrogen atom, Rx is not a phenyl group, and Rz is an aryl group substituted or not substituted with one or more lower alkyl groups.

Formamides forming a feature of the invention are: N-(α-adamantyl-idene-4-toluenesulphonylmethyl)-formamide, 1-formamido-2-(4-nitrophenyl)-1-(4-toluenesulphonyl)-ethene, 1-formamido-2,2-diphenyl-1-(4-toluenesulphonyl)-ethene, 1-formamido-1-(4-toluenesulphonyl)-ethene, 1-formamido-2-(2-furanyl)-1-(4-toluenesulphonyl)-ethene, 1-formamido-2-(4-chlorophenyl)-1-(4-toluenesulphonyl)-

ethene, 1-formamido-2-phenyl-1-(4-toluenesulphonyl)-1-propene, 1-formamido-2-(2-furanyl)-1-(4-toluenesulphonyl)-1-propene, N-tetrahydropyran-4-ylidene-(4-toluenesulphonyl)-methylformamide, 1-formamido-1-(4-toluenesulphonyl)-1-nonene, N-/-α-(thian-4-ylidene)-4-toluenesulphonylmethyl/-formamide, 1-formamido-2-methyl-2-(thien-2-yl)-1-(4-toluenesulphonyl)-1-propene, N-(α-cycloheptylidene-4-toluenesulphonylmethyl)-formamide, 1-formamido-2-(2-thienyl)-1-(4-toluenesulphonyl)-ethene, N-/-α-(1-methylpiperid-4-ylidine)-4-toluenesulphonylmethyl/-formamide, 2-phenyl-1-formamido-1-(4-toluenesulphonyl)-1-hexene, N-(α-cyclooctylidene-4-toluenesulphonylmethyl)-formamide, 1-formamido-2-methyl-1-(4-toluenesulphonyl)-1-undecene, 1-formamido-2-(4-nitrophenyl)-1-(4-toluenesulphonyl)-1-propene, 1-formamido-2-(4-methoxyphenyl)-1-(4-toluenesulphonyl)-ethene, 1-formamido-1(4-toluenesulphonyl)-1-pentene, N-(α-cyclohexylidenebenzenesulphonylmethyl)-formamide, 1-formamido-2-pentyl-1-(4-toluenesulphonyl)-1-heptene, 2-(5-chloro-2-thienyl)-1-formamido-1-(4-toluenesulphonyl)-1-propene, 1-formamido-2-(4-pyridyl)-1-(4-toluenesulphonyl)-ethene, N-/-α-(3-cholestanylidene)-4-toluenesulphonylmethyl/-formamide, 1-formamido-2-methyl-1-(4-toluenesulphonyl)-4-(1,1,3-trimethylcyclohex-2-en-2-yl)-3-trans-1,3-butadiene, 2-/-1-formamido-1-(4-toluenesulphonyl)-ethen-2-yl/-methylpyrrole, 2-(4-phenylphenyl)-1-formamido-1-(4-toluenesulphonyl)-1-propene. These compounds are important intermediates for the formation of the compounds having formula (I) or (II), according to the invention.

By-products may be formed when reacting toluenesulphonylmethylisocyanide with certain aldehydes, for example with octanal, resulting easily in 4-toluenesulphonyl-5-heptyl-2-oxazoline, or with β-ionone, resulting easily in 3-acetyl-4-(1,1,3-trimethylcyclohex-2-en-2-yl)-pyrrole. These compounds are also a feature of the invention since they may have useful fungicidal properties.

The compounds of formula (I) or (II), according to the invention, in which Rx and Ry do not represent hydrogen atoms, are generally stable, and may generally be stored at room temperature. Those compounds in which Rx or Ry is a hydrogen atom should be stored generally at lower temperatures, e.g. in a refrigerator. The 1-isocyano-1-p-toluenesulphonylethene (Rx and Ry hydrogen atoms) appears to be very unstable. Notwithstanding this the compound, in the reaction medium in which it must be present, could be converted with a primary amine into the corresponding imidazole, which in turn,

may be converted into its picrate. This proves that 1-isocyano-1-p-toluenesulphonylethene exists.

Theoretically two isomers of the compounds of formula (I) or (II), according to the invention, may be formed. In many cases only one isomer is found but in a few cases E and Z isomers have been found.

For the preparation of the imidazoles from the compounds having formula (I), according to the invention an amine or ammonia is used. Two equivalents of the primary amine or ammonia are added to a solution of the compound, and a fast exothermic reaction follows, resulting in the imidazole in question. The reaction is very slow if aromatic amines, such as aniline, are used.

The compounds according to the invention may be used for the formulation of pharmaceutic preparations, such as ointments and tablets for local, e.g. vaginal, use or for injection liquids. The active compound is mixed in the usual manner with the formulating components useful for that purpose.

The invention is illustrated by the following examples.

Example 1

A. Preparation of 1-isocyano-2-phenyl-1-(4-toluenesulphonyl)-ethene. To a solution of 7.53 g (25 mmoles) of the compound prepared according to Example 32 in 25 ml of dry 1.2-dimethoxyethane 17.5 ml (125 mmoles) of triethylamine was added at once at -5°C, followed by an addition of 2.5 ml (27.3 mmoles) of phosphorusoxychloride ($POCl_3$) in 2.5 ml of 1,2-dimethoxyethane in about 5 minutes at -10 to -5°C. After stirring for 1 hour at 0°C the mixture was added to 500 ml of iced water. Immediately the mixture was extracted with dichloromethane, dried with magnesium sulphate, concentrated and treated with methanol (30 ml, -20°C). A yield of 3.67 g (54%) of the title compound was obtained, having a melting point of 80°C (dec.). Crystallisation from methanol resulted in an analytically pure sample with the same melting point.

IR spectrum (nujol, $cm^{-1}$): 2190; 1630; 1165; 1145.

PMR spectrum (100 Mc, $CDCl_3$, $\delta$ in ppm): 2.44 (s, 3H); 7.38 (d, 2H); 7.4 (m, 5H); 7.67 (s, 1H); 7.88 (d, 2H).

$^{13}$C-NMR spectrum ($CDCl_3$, $\delta$ in ppm, $^{-1}J_{C-H}$ indicated alone): 21.0 (q, 127 c); 123.6 (s); 128.4 (d, about 165 c); 128.5 (d, about 165 c); 129.1 (s); 129.6 (d, about 165 c); 130.0 (d, about 165 c); 131.9 (d, about 155 c); 132.9 (s); 134.4 (d, about 160 c); 145.4 (s);

178.5 (s).

Analysis calculated for $C_{16}H_{13}NO_2S$: C 67.82; H 4.62; N 4.94; S 11.29; found C 67.5; H 4.7; N 5,0; S 11.2%.

B. Preparation of 1-t-butyl-5-phenylimidazole.

t-Butylamine (150 mg, 2.05 mmoles) was added to a solution of 283 mg (1.00 mmole) of the compound prepared according to Example 1A in 5 ml of methanol and the mixture was stirred for 24 hours at room temperature. The mixture was diluted with water and extracted with dichloromethane. The extract was dried with magnesium sulphate, concentrated and sublimated (0.2 mm Hg, bath temperature 80 to 100°C). A yield of 165 mg (82%) of 1-t-butyl-5-phenylimidazole was obtained, having a melting point of 103 to 109°C. Repeated sublimation if vacuo resulted in an analytically pure sample having a melting point of 110 to 112°C.

IR spectrum (nujol, $cm^{-1}$): 3200; 1475; 1385; 1238; 1130; 925; 775; 715.

PMR spectrum (60 Mc, $CDCl_3$, $\delta$ in ppm): 1.42 (s, 3H); 6.38 (d, J = 1 c, 1H); 7.3 (m, 5H); 7.59 (d, J = 1 c, 1H).

Analysis calculated for $C_{13}H_{16}N_2$: C 77.96; N 13.99; found: C 78.1; H 8.1; N 14.1.

C. Preparation of 1-cyclohexyl-5-phenylimidazole.

In the way as indicated in Example 1B the above-indicated compound was prepared from 1.10 g (11 mmoles) of cyclohexylamine, 1.42 g (5.0 mmoles) of the compound prepared according to Example 1A and 25 ml of methanol. The weakly exothermic reaction (temperature increase to about 30°C) was, according to TLC, finished after 30 minutes. By sublimation in vacuo (twice, 0.1 mm Hg, bath temperature 80 to 140°C) resulted in a solidifying oil. Yield 1.1 g (97%) of 1-cyclo-hexyl-5-phenylimidazole with a melting point of about 50°C; melting point of the picrate (crystallised from ethanol) 150 to 153°C.

IR spectrum (nujol, $cm^{-1}$): 3150; 1480; 1465; 1385; 1230; 1125; 775; 710.

PMR spectrum ($CCl_4$, $\delta$ in ppm): 1.0-2.3 (m, 10 H); 3.9 (br.s, 1H); 6.82 (d, J = about 1 c, 1H); 7.30 (m, 5H); 7.45 (d, J = about 1 c, 1H).

Analysis calculated for the picrate $C_{21}H_{21}N_5O_7$: C 55.38;

H 4.65; N 15.38; found: C 55.1; H 4.5; N 15.4.

D. Other imidazoles.

In the way as indicated in Examples 1B and 1C there were prepared: 5-phenylimidazole (reaction time 3 hours; yield 65%; melting point 118 to 122°C; melting point of picrate 217°C);

1-methyl-5-phenylimidazole (reaction time 5 minutes; yield 87%; melting point 90 to 94°C9.

## Example 2

Preparation of α-adamantylidene-α-(4-toluenesulphonyl) methylisocyanide.

A solution of 4.9 g (25 mmoles) of p-toluenesulphonylmethyl isocyanide in 15 ml of dry 1,2-dimethoxyethane was added dropwise to a stirred suspension of 4.0 g (about 35 mmoles) of potassium t-butylate in a nitrogen atmosphere at -30 to -10°C. Then a solution of 3.75 g (25 mmoles) of adamantone in 5 ml of 1,2-dimethoxyethane was added at -40 to -30°C. After stirring for 30 minutes at -30°C 2.4 g (40 mmoles) of acetic acid in 10 ml of 1,2-dimethoxyethane were added, followed by 20.1 ml (145 mmoles) of triethylamine. A solution of 5 ml (50 mmoles) of phosphorus oxychloride in 10 ml of 1,2-dimethoxyethane was added between -10 and -5°C. After stirring for an hour at 0°C, followed by the recovery as indicated in Example 1, a yield of 5.21 g (64%) of the title compound was obtained. Melting point 139 to 140°C. An analytically pure sample was obtained by crystallisation from ethanol. Melting point 140.5 to 141.5°C.

IR spectrum (nujol, $cm^{-1}$): 2190; 1610; 1355; 1170.

PMR spectrum (60 Mc, $CDCl_3$, δ in ppm): 1.4-2.2 (m, 12H); 2.08 (br.s, 1H); 2.44 (s, 3H); 4.35 (br.s, 1H); 7.38 (d, 2H); 7.88 (d, 2H).

Analysis calculated for $C_{19}H_{21}NO_2S$ (%): C 69.69; H 6.46; N 4.28; S 9.79; found C 69.5; H 6.5; N 4.3; and S 9.7.

## Example 3

A. Preparation of 1-isocyano-2-(4-nitrophenyl)-1-(4-toluenesulphonyl)-ethene.

Triethylamine (8.5 ml. 60 mmoles) was added to a suspension of 3.46 g (10 mmoles) of the compound prepared according to Example 34 in 30 ml of 1,2-dimethoxyethane at -10°C. Then 1.2 ml (13 mmoles) of phosphorus oxychloride in 10 ml of 1,2-dimethoxyethane were added dropwise at -5°C, and the mixture was stirred for

5 hours at 0°C. It was poured into 500 ml of iced water, extracted with dichloromethane and treated with magnesium sulphate and a small amount of activated carbon. It was concentrated to 100 ml, filtered through a layer (3 cm) of alumina (Merck, act. II-III) and concentrated. Then the residue was treated with 50 ml of methanol at -20°C, resulting in a yield of 1.80 g (55%) of the greenish above-indicated compound having a melting point of 130°C (decomp.). Crystallisation from acetone resulted in an analytically pure sample with the same melting point.

IR spectrum (nujol, $cm^{-1}$): 2170; 1640; 1535; 1360; 1170.

PMR spectrum (60 Mc, $CDCl_3$, $\delta$ in ppm): 2.48 (s, 3H); 7.40 (d, 2H); 7.73 (s, 1H); 7.87 (d, 2H); 7.88 (d, 2H); 8.27 (d, 2H).

$^{13}C$-NMR spectrum ($CDCl_3$, $\delta$ in ppm): 21.6; 124.1; 129.2; 130.1; 130.3; 131.2; 131.8; 132.4; 135.5; 146.7; 149.1; 181.

Analysis calculated for $C_{16}H_{12}N_2O_4S$: C 58.53; H 3.68; N 8.53; S 9.76%; found C 58.7; H 3.9; N 8.4 and S 9.4%.

B. Preparation of 1-methyl-5-p-nitrophenylimidazole.

The above-indicated compound was prepared in the way as described in Examples 1B and 1C in a yield of 88%. The reaction time was 3 minutes. Melting point 163 to 165°C.

## Example 4

A. Preparation of 3,3-dimethyl-1-isocyano-1-(4-toluenesulphonyl)-1-butene.

In the way as described in Example 1A this compound was prepared from 2.81 g (10 mmoles) of N-(1-toluenesulphonyl-3,3-dimethyl-1-butenyl)formamide in a yield of 2.03 g (77%). Melting point 83-88°C. An analytically pure sample was obtained from a mixture of diethylether and petroleum ether (boiling track 40 to 60°C). That sample had a melting point of 87 to 88°C.

IR spectrum (nujol, $cm^{-1}$): 2190; 1635; 1350; 1770.

PMR spectrum (100 Mc, $CDCl_3$, $\delta$ in ppm): 1.24 (s, 9H); 2.45 (s, 3H); 6.88 (s, 1H); 7.38 (d, 2H); 7.82 (d, 2H).

$^{13}C$-NMR spectrum ($CDCl_3$, $\delta$ in ppm): 21.1 (q); 27.9 (q or quint., 127 c); 33.6 (s); 125.9 (br.s); 128.5 (d, 166 c); 129.7 (d, 162 c); 132.9 (s); 145.4 (s) 148.5 (d, 161 c); 177.8 (s).

Analysis calculated for $C_{14}H_{17}NO_2S$ (%): C 63.83; H 6.51; N 5.32; S 12.17; found C 64.0; H 6.5; N 5.3 and S 12.3.

The same compound was also obtained according to the

method of Example 2 from p-toluenesulphonylmethyl isocyanide in a yield of 30%.

B. Preparation of 1-methyl-5-(1,1-dimethylethyl)imidazole.

In the way as described in the Examples 1B and 1C the above-indicated compound was prepared and was obtained in the form of an oil. The yield in the form of its picrate was 46% and the melting point of the picrate was 152 to 155°C (decomp.).

Example 5

Preparation of 1-isocyano-2-methyl-1-(4-toluenesulphonyl)-1-propene.

This compound was prepared in the way as described in Example 2 from 1.45 g (25 mmoles) of acetone in a yield of 3.76 g (64%). Melting point 77 to 81°C. An analytically pure sample was obtained by crystallisation from methanol. Melting point 78 to 81°C.

IR spectrum (nujol, $cm^{-1}$): 2170; 1615; 1345; 1170.

PMR spectrum (60 Mc, $CDCl_3$. $\delta$ in ppm): 2.08 (s, 3H); 2.47 (s, 3H); 7.35 (d, 2H); 7.82 (d, 2H).

Analysis calculated for $C_{12}H_{13}NO_2S$ (%): C 61.25; H 5.57; N 5.95; S 13.62; found: C 61.1; H 5.5; N 6.0 and S 13.6.

Example 6

Preparation of 2,2-diphenyl-1-isocyano-1-(4-toluenesulphonyl)-ethene.

This compound was prepared in the way as described in Example 1A from 1.89 (5 mmoles) of the compound obtained according to Example 36 in a yield of 1.21 g (68%). Melting point 123 to 127°C (decomp.). An analytically pure sample was obtained by crystallisation from methanol. Melting point 130 to 132°C (decomp.).

IR spectrum (nujol, $cm^{-1}$): 2180; 1610; 1360; 1170.

Analysis calculated for $C_{22}H_{17}NO_2S$ (%): C 73.51; H 4.77; N 3.90; S 8.92; found: C 73.6; H 4.7; N 3.8 and S 8.9.

Example 7

Preparation of α-cyclohexylidene-α-(4-toluenesulphonyl)-methylisocyanide.

This compound was prepared in the way as described in Example 2 from 2.5 g (25 mmoles) of cyclohexanone in a yield of 3.96 g (58%). Melting point 115°C (decomp.). An analytically pure sample was obtained by crystallisation from methanol. The melting point remained unchanged.

IR spectrum (nujol, $cm^{-1}$): 2180; 1610; 1345; 1165.

PMR spectrum (60 Mc, $CDCl_3$, δ in ppm): 1.63 (m, 6H); 2.44 (s, 3H); 2.45 (m, 2H); 2.90 (m, 2H); 7.38 (d, 2H); 7.88 (d, 2H).

Analysis calculated for $C_{15}H_{17}NO_2S$ (%): C 65.43; H 6.22; N 5.09; S 11.64; found: C 65.0; H 6.2; N 5.0 and S 11.6.

Example 8

Preparation of 1-isocyano-1-(4-toluenesulphonyl)-ethene and of 1-methyl-imidazole therefrom.

1.2 ml (12 mmoles) of $POCl_3$ was added to a solution of 1.13 g (5.0 mmoles) of the compound according to Example 38 in 10 ml of dry 1,2-dimethoxyethane at -30°C. Then 3.8 ml (20 mmoles) of triethylamine in 2.5 ml of 1,2-dimethoxyethane was added slowly (during 10 minutes) to the solution at -30°C, and the mixture was stirred for 1.5 hours at -5 to -10°C. The mixture was added to saturated $NaHCO_3$ and extracted with dichloromethane and concentrated to form an impure brown oil. The oil was treated with an excess of methylamine in the way as described in Example 1B to form a low yield of 1-methylimidazole which was identified as its picrate. Melting point of the picrate 153 to 156°C. The formation of 1-methylimidazole is an indication that 1-isocyano-1-(4-toluenesulphonyl) ethene was present in the impure brown oil.

Example 9

Preparation of α-cyclopentylidene-α-(4-toluenesulphonyl)-methylisocyanide.

A solution of 9.75 g (50 mmoles) of p-toluenesulphonylmethylisocyanide in 50 ml of 1,2-dimethoxyethane was added dropwise to a stirred suspension of 5.6 (50 mmoles) of potassium t-butylate in 100 ml of 1,2-dimethoxyethane in a nitrogen atmosphere at -60 to -40°C. The mixture obtained was cooled to -70°C, and a solution of 4.2 g (50 mmoles) of cyclopentanone in 25 ml of 1,2-dimethoxyethane was added dropwise at -60 to -40°C. After stirring for 15 minutes at -30°C 2.86 ml (50 mmoles) of acetic acid were added and the mixture was concentrated in vacuo. The residue was dissolved in 500 ml of dichloromethane, washed with water, dried and concentrated in vacuo. The residue was dissolved in 25 ml of 1,2-dimethoxyethane and 35 ml of triethylamine were added to the mixture. 5 ml (50 mmoles) of phosphorusoxychloride in 6 ml of 1,2-dimethoxyethane were added between -30 and -10°C. After stirring for an hour at -20°C and recovery as described in Example 1A a yield of 9.0 g (70%) of the

above-mentioned compound was obtained. Crystallisation from a mixture of ethanol and hexane resulted in a pure sample having a melting point of 108°C.

IR spectrum (KBr, $cm^{-1}$): 2130; 2100; 1618; 1610; 1336; and 1153.

PMR spectrum (60 Mc, $CDCl_3$, $\delta$ in ppm): 1.70 (m, 4H); 2.44 (s, 3H); 2.63 (m, 2H); 2.99 (m, 2H); 7.4 (d, 2H) and 7.88 (d, 2H).

Example 10

Preparation of 2-(2-furyl)-1-isocyano-1-(4-toluenesulphonyl)-ethene.

A solution of 6.44 g (22.1 mmoles) of the compound prepared according to Example 39 and 17.5 ml of triethylamine in 12.5 ml of 1,2-dimethoxyethane was cooled to -40°C. A solution of 2.5 ml of phosphorusoxychloride in 3 ml of 1,2-dimethoxyethane was added below -30°C. After stirring for 15 minutes at -30°C the mixture was poured into 200 ml of iced water. The mixture obtained was extracted with dichloromethane and the extract was dried and concentrated. The residue was dissolved in ethanol, treated with activated carbon and concentrated in vacuo. After chromatography over neutral alumina (4 x 25 cm) with dichloromethane a yield of 2.1 g (35%) of a pale yellow compound was obtained; melting point 95°C.

IR spectrum (KBr, $cm^{-1}$): 2103; 1635; 1617; 1597; 1338; 1160.

PMR spectrum (60 Mc, $CDCl_3$, $\delta$ in ppm): 2.47 (s, 3H); 6.67 (2x d, 1H, J = 4 and 2 c); 7.22 (d, 1H, J = 4 c); 7.44 (d, 2H); 7.63 (s, 1H); 7.72 (d, 1H, J = 2 c); 7.96 (d, 2H).

Example 11

Preparation of 1-isocyano-2-(4-chlorophenyl)-1-(4-toluenesulphonyl)-ethene.

In the way as described in Example 1A this compound was prepared from 10 g of the product according to Example 40 in a yield of 8 g. After crystallisation from methanol the analytically pure compound was obtained in a yield of 65%, with a melting point of 133°C.

IR spectrum (KBr, $cm^{-1}$): 2120; 1620; 1590; 1340; 1160.

PMR spectrum (60 Mc, $CDCl_3$, $\delta$ in ppm): 2.47 (s, 3H); 7.2-8.1 (m, 9H).

Example 12

Preparation of 2-phenyl-1-isocyano-1-(4-toluenesulphonyl)-1-propene.

This compound was obtained from the compound according to Example 41 and was prepared according to the method of Example 18, except that N-methyl-morpholine was used instead of triethylamine. After recovery the compound was chromatographed over aluminiumoxide (act. II-III). The oil which was obtained was crystallised from methanol. Yield 15%. TLC: toluene/ethyl acetate 9:1 Rf = 0.65. Melting point 74°C.

PMR-spectrum (60 MHz, $CDCl_3$, δ in ppm): 2.42 (s, 3H); 2.56 (s, 3H); 7.24 (s, 5H); 7.34 (d, 2H); 7.86 (d, 2H).

IR-spectrum (KBr, $cm^{-1}$): 2101; 1685; 1592; 1491; 1330; 1166.

Example 13

Preparation of 1-isocyano-2-(2-furanyl)-1-(4-toluenesulphonyl)-1-propene.

This compound was obtained from the compound according to Example 42 and was prepared according to the method of Example 18. After recovery the compound was crystallised twice from methanol. Yield 31%. TLC: toluene/ethyl acetate 9:1 Rf = 0.54. Melting point 75°C.

PMR-spectrum (60 MHz, $CDCl_3$, δ in ppm): 2.47 (s, 3H); 2.61 (s, 3H) 6.65 (q, 1H); 7.40 (d, 1H); 7.45 (d, 2H); 7.68 (d, 1H); 7.98 (d, 2H).

IR-spectrum (KBr, $cm^{-1}$): 2107; 1578; 1340; 1157.

Example 14

Preparation of α-(tetrahydropyran-4-ylidene)-α-(4-toluene-sulphonyl)-methyl-isocyanide (a) and α-(2,3-dihydro-6H-pyran-4-yl)-α-(4-toluenesulphonyl)-methylisocyanide (b).

Compound (a) was obtained from the compound according to Example 43 and was prepared according to the method of Example 18. In order to avoid moving of the double bond N-methylmorpholine was used instead of triethylamine. After recovery the compound was crystallised from methanol. Yield 70%. TLC: toluene/ethyl acetate 9:1 Rf = 0.39. Melting point 104°C.

Compound (b) was obtained by using the method of Example 18, i.e. with triethylamine. After recovery the compound was crystallised from methanol. Yield 67%. TLC: toluene/ethyl acetate 9:1 Rf = 0.29. Melting point 114°C.

PMR spectrum compound (a) (60 MHz, $CDCl_3$, δ in ppm): 2.48

(s, 3H); 2.61 (t. 2H); 3.24 (t, 2H); 3.82 (m, 4H); 7.48 (d, 2H); 7.95 (d, 2H).

IR-spectrum compound (a) (KBr, $cm^{-1}$): 2127; 2088; 1607; 1598; 1335; 1160; 1097.

PMR-spectrum compound (b) (60 MHz; $CDCl_3$, $\delta$ in ppm): 2.35 (m, 2H); 2.48 (s, 3H); 3.80 (t, 2H); 4.20 (m, 2H); 5.03 (s, 1H); 6.0 (m, 1H); 7.47 (d, 2H); 7.91 (d, 2H).

IR-spectrum compound (b) (KBr, $cm^{-1}$): 2146; 1599; 1335; 1160.

## Example 15

Preparation of 1-isocyano-1-(4-toluenesulphonyl)-1-nonene.

This compound was prepared from the formamide according to Example 44 in the way as described in Example 18. After recovery the oil obtained was purified over aluminium oxide (act. II-III). Yield 25%. TLC toluene/ethyl acetate 9:1 Rf = 0.78. The final product was still an oil.

PMR-spectrum (60 MHz, $CDCl_3$, $\delta$ in ppm): 0.89 (t. 3H); 1.28 (broad s, 10 H); 2.32 (t. 2H); 2.45 (s, 3H); 7.03 (t, 1H); 7.37 (d, 2H); 7.82 (d, 2H).

IR spectrum (KBr, $cm^{-1}$): 2135, 2112; 1628; 1598; 1339; 1161.

## Example 16

Preparation of α-(thian-4-ylidene)-α-(4-toluenesulphonyl)-methylisocyanide (a) and α-(3,4-dehydrothian-4-yl)-α-(4-toluenesulphonyl)-methylisocyanide.

Compound (a) was prepared from the formamide according to Example 45 in the way as described in Example 18, using N-methylmorpholine instead of triethylamine to avoid moving of the double bond. After pouring into water the solid which was formed was filtered off. Yield after drying 90%. TLC toluene/ ethyl acetate 9:1 Rf = 0.70.

Compound (b) was prepared according to the method of Example 18, thus using triethylamine. However, a mixture of compound (a) and (b) in the ratio of 1:4 was obtained. Another experiment was carried out, using 1,4-diazabicyclo-(2,2,2)-octane which is a stronger base than triethylamine.

A solution of 15 mmoles of the formamide (Example 45) in 25 ml of dimethoxyethane was cooled to -70°C. With stirring a suspension of 56.25 mmoles of 1,4-diazabicyclo-(2,2,2)-octane in 50 ml

dimethoxyethane was added at that temperature. Then a solution of 22.5 mmoles $POCl_3$ in 10 ml of dimethoxyethane was added dropwise in 10 minutes at -60°C. After stirring for 1 hour at -60°C and 1 hour at 0°C another 22.5 mmoles of $POCl_3$ were added and the mixture was stirred for 1 hour at room temperature. Then the mixture was cooled to -40°C and 225 mmoles of triethylamine were added. After keeping the mixture for 1 hour at -10°C the reaction had proceeded for about one half. After recovery the oil obtained was dissolved in 20 ml of dimethoxyethane and cooled to -60°C. After addition of 75 mmoles of triethylamine a solution of 30 mmoles $POCl_3$ in 5 ml of dimethoxyethane was added dropwise in 10 minutes. After stirring for half an hour the reaction was finished. After recovery the oil was dissolved in 20 ml methylene chloride and cooled to 0°C. Then 20 mmoles of 1,4-diazabicyclo-(2,2,2)-octane was added and the mixture was stirred for 15 minutes, followed by another 20 mmoles of 1,4-diazabicyclo-(2,2,2)-octane. The mixture was washed to neutral and the oil obtained was crystallised from methanol. Yield 36%, containing only 10% of compound (a). TLC toluene/ethyl acetate 9:1 Rf = 0.54. Melting point 90°C.

PMR-spectrum compound (a) (60 MHz, $CDCl_3$, $\delta$ in ppm): 2.49 (s, 3H); 2.80 (m, 6H); 3.28 (m, 2H); 7.48 (d, 2H); 7.93 (d, 2H).

IR-spectrum compound (a) (KBr, $cm^{-1}$): 2125; 2097; 1600; 1338; 1160.

PMR-spectrum compound (b) (60 MHz, $CDCl_3$, $\delta$ in ppm): 2.50 (s, 3H); 2.4-3.0 (m, 4H); 3.25 (m, 2H); 5.01 (s, 1H); 6.12 (t, 1H); 7.50 (d, 2H); 7.95 (d, 2H).

IR-spectrum compound (b) (KBr, $cm^{-1}$): 2140; 1595; 1330; 1160.

## Example 17

Preparation of 1-isocyano-1-(4-toluenesulphonyl)-2-(2-thienyl)-1-propene.

Using the formamide according to Example 46 the preparation of this compound was carried out as in Example 18. After recovery the compound was crystallised from methanol. Yield 25%. TLC toluene/ethyl acetate 1:1 Rf = 0.62. Melting point 92°C.

PMR spectrum (60 MHz, $CDCl_3$, $\delta$ in ppm): 2.48 (s, 3H); 2.71 (s, 3H); 7.21 (double d, 1H); 7.68 (double d, 1H); 7.82 (double d, 1H); 7.98 (d, 2H); 8.01 (d, 2H).

IR-spectrum (KBr, $cm^{-1}$): 2092; 1636; 1601; 1563; 1320; 1150.

Example 18

Preparation of α-cycloheptylidene-α-(4-toluenesulphonyl)-methylisocyanide (a) and α-(cyclohepten-1-yl)-α-(4-toluenesulphonyl)-methylisocyanide (b).

Using the formamide according to Example 47 the preparation of compound (a) was carried out as in this Example for compound (b), except that N-methyl-morpholine was used instead of triethylamine. Yield 81%. TLC toluene/ethyl acetate 1:1 Rf = 0.9. Melting point 78°C.

Compound (b) was prepared as follows: To a solution of 4.52 g (14.7 mmoles) of the formamide according to Example 47 in 10 ml of dimethoxyethane, cooled to -70°C, 10.3 ml of triethylamine (74 mmoles) were added. Then a solution of 1.5 ml (16.5 mmoles) $POCl_3$ in 2 ml of dimethoxyethane were added dropwise at -60°C. The reaction was finished after half an hour according to TLC. Iced water (50 ml) was added at -20°C, resulting in the formation of an oil. The reaction mixture was extracted with two portions of 100 ml methylene chloride. The methylene chloride was dried with $MgSO_4$ and was evaporated subsequently. Yield 4.2 g of a solid, which was crystallised from 10 ml boiling ethanol. After addition of 20 ml of n-hexane the compound crystallised. The mixture was stored overnight in a refrigerator and filtered off. It was washed with cold n-hexane. After drying the yield was 2.2 g (52%). TLC toluene/ethyl acetate 1:1 Rf = 0.85; toluene/ethyl acetate 9:1 Rf = 0.70. Melting point 97°C.

PMR spectrum compound (a) (60 MHz, $CDCl_3$, δ in ppm): 1.56 (m, 8H); 2.44 (s, 3H); 2.60 (t, 2H); 3.00 (t, 2H); 7.42 (d, 2H); 7.90 (d, 2H).

IR spectrum compound (a) (KBr, $cm^{-1}$): 2109; 1596; 1583; 1331; 1160.

PMR spectrum compound (b) (60 MHz, $CDCl_3$, δ in ppm): 1.18 (broad s, 6H); 2.30 (m, 4H); 2.47 (s, 3H); 4.96 (s, 1H); 6.03 (t, 1H); 7.32 (d, 2H); 7.88 (d, 2H).

IR spectrum compound (b) (KBr, $cm^{-1}$): 2139; 1598; 1337; 1158.

Example 19

Preparation of 2-phenyl-1-isocyano-1-(4-toluenesulphonyl)-1-hexene.

Triethylamine (7.2 ml, 25.8 mmoles) was added dropwise to

a solution of 8.62 mmoles of the formamide according to Example 50 in 9 ml of dimethoxyethane at -10°C. After stirring at 0°C for an hour the reaction mixture was poured into 450 ml of iced water. The mixture was extracted with methylene chloride and the extract was dried and evaporated, resulting in 3 g of a solid. The solid was dissolved in 3 ml of methanol and stored overnight at -20°C. The solid thus obtained was filtered of. Yield 57.5%. TLC toluene/ethyl acetate 9:1 Rf = 0.68. Melting point 78°C. The product was a mixture of the E and Z isomers.

PMR spectrum (60 MHz, $CDCl_3$, δ in ppm): 0.87 (t, 3H); 1.31 (m, 4H); 2.46 (s, 3H); 3.08 (t, 2H); 7.15-8.05 (m, 9H).

IR spectrum (KBr, $cm^{-1}$): 2130; 1597; 1345; 1170.

Example 20

Preparation of α-(cycloocten-1-yl)-α-(4-toluenesulphonyl) methylisocyanide.

To a solution of 25 mmoles of the formamide according to Example 51 in 75 ml of dimethoxyethane 20 ml of triethylamine (150 mmoles) were added dropwise at -10°C, followed by 3 ml $POCl_3$ at -5°C. After stirring at -5°C for 75 minutes the reaction mixture was poured into 500 ml of iced water and the mixture was extracted with methylene chloride. The extract was treated with activated carbon, and was dried and evaporated. The residue was taken up in 5 ml methanol. The mixture was stored for several days at -20°C, and then the precipitate was filtered off. Yield 39%. TLC toluene/ethyl acetate 9:1 Rf = 0.7. Melting point 70 to 73°C.

PMR spectrum (60 MHz, $CDCl_3$, δ in ppm): 1.54 (m, 8H); 2.35 (m, 4H); 2.50 (s, 3H); 5.04 (s, 1H); 5.98 (t, 1H); 7.48 (d, 2H); 7.93 (d, 2H).

IR spectrum (KBr, $cm^{-1}$): 2150; 1595; 1330; 1155.

Example 21

Preparation of 1-isocyano-2-methyl-1-(4-toluenesulphonyl)-1-undecene.

A. Preparation of the E-isomer.

To a mixture of 32.6 mmoles of E-1-formamido-2-methyl-1-(4-toluenesulphonyl)-1-undecene (cf. Example 52), 97.8 mmoles of N-methylmorpholine and 100 ml dimethoxyethane a solution of 42.4 mmoles $POCl_3$ in 33 ml of dimethoxyethane was added dropwise at -5°C. After stirring at 0°C for 90 minutes the reaction mixture was treated as usual (cf. Example 19). Since a considerable amount of the starting

material was still present the compound was treated once again, resulting in a complete conversion. The reaction mixture was poured into water and extracted with methylene chloride. The extract was dried and evaporated. The residue was treated with methanol at a temperature of -20°C. The solid was filtered of in the cold (the compound is an oil at room temperature). A second lot was obtained from the mother liquor. Yield 49%. TLC toluene/ethyl acetate 3:1 Rf = 0.87.

PMR-spectrum (60 MHz, $CDCl_3$, δ in ppm): 0.87 (t, 3H); 1.23 (m, 14H); 2.3 (m, 2H); 2.27 (s, 3H); 2.44 (s, 3H); 7.36 (d, 2H); 7.85 (d, 2H).

IR-spectrum ($CHCl_3$, $cm^{-1}$): 2103; 1599; 1336; 1153.

B. Preparation of the Z-isomer.

To a mixture of 8.2 mmoles of Z-1-formamido-2-methyl-1-(4-toluenesulphonyl)-1-undecene (cf. Example 52), 2.75 ml of N-methylmorpholine and 25 ml of dimethoxyethane a solution of 12.3 mmoles $POCl_3$ in 9 ml of dimethoxyethane was added dropwise at -5°C. As in preparation A the mixture had to be treated twice. After pouring into water, extracting with methylene chloride, etc. an oil was obtained which was triturated with methanol of -10°C. The solid was filtered off and the mother liquor was treated, resulting in a yield of 50%. TLC toluene/ethyl acetate 3:1 Rf = 0.87. Melting point 38 to 43°C.

PMR spectrum (60 MHz, $CDCl_3$, δ in ppm): 0.90 (t, 3H); 1.27 (m, 14H); 2.03 (s, 3H); 2.44 (s, 3H); 2.72 (t, 2H); 7.33 (d, 2H); 7.81 (d, 2H).

IR spectrum ($CHCl_3$, $cm^{-1}$): 2101; 1595; 1339; 1151.

## Example 22

Preparation of 1-isocyano-2-(4-nitrophenyl)-1-(4-toluenesulphonyl)-1-propene.

To a mixture of 33 mmoles of 1-formamido-2-(4-nitrophenyl)-1-(4-toluenesulphonyl)-1-propene (cf. Example 53), 0.2 mole of N-methylmorpholine and 100 ml of dimethoxyethane a solution of 6.1 ml $POCl_3$ in 35 ml of dimethoxyethane was added dropwise at -5°C. The mixture was stirred for 90 minutes and poured into 350 ml of iced water. The mixture was extracted with methylene chloride and the extract was washed with iced water, dried and evaporated. The residue was mixed with 25 ml of methanol and stored overnight at -20°C. The solid was filtered off. Yield 76%. The product appeared to consist of a mixture of the title compound and 20% of 1-isocyano-2-(4-nitrophenyl)-1-(4-toluenesulphonyl)-2-propene. The occurance of the two compounds was proved by PMR and by TLC (toluene): the Rf for the title compound = 0.14 and that of the by-product 0.10. Melting point 140°C.

PMR-spectrum (title compound, 60 MHz, $CDCl_3$/DMSO, δ in ppm): 2.47 (s, 3H); 2.63 (s, 3H); 7.3 to 8.4 (m, 8H). Characteristic signals of the by-product: 5.66 (s, 1H); 5.81 (s, 1H); 5.97 (s, 1H).

IR-spectrum (KBr, $cm^{-1}$): 2142; 2106; 1599; 1517; 1350; 1340; 1160.

Example 23

Preparation of 1-isocyano-2-(4-methoxyphenyl)-1-(4-toluenesulphonyl)-ethene.

To a mixture of 45 mmoles of 1-formamido-2-(4-methoxyphenyl)-1-(4-toluenesulphonyl)-ethene (cf. Example 54), 270 mmoles of N-methylmorpholine and 135 ml of dimethoxyethane a solution of 8.2 ml $POCl_3$ in 45 ml of dimethoxyethane was added dropwise at -5°C. The mixture was stirred for 90 minutes. The mixture was poured into water and extracted with methylene chloride. The extract was dried and evaporated. The semi-solid residue was triturated with a mixture of 100 ml of hexane and 25 ml of methanol. Yield 11%. TLC toluene/ethyl acetate 3:1 Rf = 0.76. Melting point 95°C.

PMR spectrum (60 MHz, $CDCl_3$, δ in ppm): 2.44 (s, 3H); 3.84 (s, 3H); 6.97 (d, 2H); 7.40 (d, 2H); 7.63 (s, 1H); 7.80 (d, 2H); 7.92 (d, 2H).

IR spectrum (KBr, $cm^{-1}$): 2850; 2110; 1605; 1335; 1155.

Example 24

Preparation of 1-isocyano-1-(4-toluenesulphonyl)-1-pentene.

To a mixture of 22.4 mmoles of 1-formamido-1-(4-toluenesulphonyl)-1-pentene (cf. Example 55), 134 mmoles of N-methylmorpholine and 25 ml of dimethoxyethane a solution of 44.8 mmoles $POCl_3$ in 5 ml of dimethoxyethane was added dropwise at -5°C. The mixture was stirred for an hour and poured into 350 ml of iced water. The mixture was then extraced with methylene chloride and the extract was washed with iced water, treated with activated carbon, dried and evaporated. The residue was chromatographed over aluminium oxide II (3x25 cm) with methylene chloride as the eluent. Yield 34% of an oil. TLC toluene/ethyl acetate 6:1 Rf = 0.67.

PMR spectrum (60 MHz, δ in ppm): 0.95 (t, 3H); 1.5 (m, 2H);

2.28 (t, 2H); 2.42 (s, 3H); 7.0 (d, 2H); 7.92 (d, 2H).

IR spectrum (KBr, $cm^{-1}$): 2131; 2108; 1629; 1595; 1338; 1167; 1148.

Example 25

Preparation of α-cyclohexylidene-α-benzenesulphonylmethylisocyanide.

To a mixture of 14.7 mmoles of N-(α-cyclohexylidene-benzenesulphonylmethyl)-formamide (cf. Example 56), 74.3 mmoles of N-methylmorpholine and 15 ml of dimethoxyethane a solution of 29.7 mmoles $POCl_3$ in 2 ml of dimethoxyethane was added dropwise at -5°C. The mixture was stirred for 90 minutes at 0°C and then the reaction mixture was poured into iced water and extracted with methylene chloride. The extract was treated with activated carbon, dried and evaporated. The residue was tirturated with 5 ml of methanol at 0°C. Yield 58%. TLC toluene/ethyl acetate 9:1 Rf = 0.6. Melting point 52 to 53.5°C.

It is remarked that by using triethylamine instead of the N-methylmorpholine isomeration of the double bond occured, and α-(cyclohexen-1-yl)-benzenesulphonylmethylisocyanide was formed.

PMR spectrum (60 MHz, $CDCl_3$, δ in ppm): 1.63 (m, 6H); 2.50 (m, 2H); 2.93 (m, 2H); 7.5 to 8.2 (m, 5H).

IR spectrum (KBr, $cm^{-1}$): 2110; 2090; 1601; 1581; 1447; 1335; 1150.

Example 26

Preparation of 1-isocyano-2-pentyl-1-(4-toluenesulphonyl)-1-heptene.

To a mixture of 40 mmoles of 1-formamido-2-pentyl-1-(4-toluenesulphonyl)-1-heptene (cf. Example 57), 240 mmoles of N-methylmorpholine and 120 ml of dimethoxyethane a solution of 7.3 ml $POCl_3$ in 40 ml of dimethoxyethane was added dropwise at -5°C. The mixture was stirred for an hour and poured into water. The mixture was extracted with methylene chloride and the extract was dried and evaporated. The residue was mixed with 7.5 ml of methanol and stored overnight at -20°C. The methanol was decanted off and the remaining oil was liberated from residual methanol by an oil pump. Yield 81%. TLC toluene/ethyl acetate 3:1 Rf = 0.67.

PMR spectrum (60 MHz, $CDCl_3$, δ in ppm): 0.87 and 0.91 (2x t, 6H); 1.35 (m, 12H); 2.35 (t, 2H); 2.44 (s, 3H); 2.72 (t, 2H); 7.38 (d, 2H), 7.87 (d, 2H).

IR spectrum (film between NaCl, $cm^{-1}$): 2120; 1595; 1342; 1162.

Example 27

Preparation of 1-isocyano-2-methyl-1-(4-toluenesulphonyl)-1-butene (a) and 1-isocyano-2-methyl-1-(4-toluenesulphonyl)-2-butene (b).

To a suspension of 16 g of potassium t-butoxide in 60 ml of dimethoxyethane a solution of 0.1 mole of (4-toluenesulphonyl)-methylisocyanide in 100 ml of dimethoxyethane was added dropwise at -30°C. The addition was followed by the addition of a solution of 0.1 mole of methylethylketone in 60 ml of dimethoxyethane at -40°C. The mixture was stirred at -30°C for 45 minutes and poured into 1200 ml of iced water. The mixture was acidified with 10 ml of glacial acetic acid and extracted with methylene chloride. The extract was dried and evaporated. The thus obtained oil (89%) contained one sole component showing an Rf of 0.30 with toluene/ethyl acetate 1:1 and 0.23 with chloroform/ether 9:1. Crystallisation from methanol, methanol/dimethylformamide, acetonitrile and dimethylformamide failed, so that the mixture was used without further purification.

To a mixture of the oil, 60 ml of triethylamine and 100 ml of dimethoxyethane a solution of 10.5 ml $POCl_3$ in 10 ml of dimethoxyethane was added dropwise at -25°C. The mixture was stirred at -30°C for 30 minutes and at 0°C for 45 minutes and an additional amount of 2.5 ml of $POCl_3$ was added. The reaction mixture was stirred for an hour, poured into 1500 ml of water and extracted with methylene chloride. The extract was washed with a saturated NaCl·solution, treated with activated carbon, dried and evaporated to 300 ml. That solution was filtered over aliminium oxide (act. II-III, 3x10 cm). The filtrate was evaporated at 0°C. The residue was mixed with 50 ml of methanol and stored for three days at -20°C. The very small amount of solid formed was filtered off and the filtrate was evaporated. The residue was mixed with 15 ml of methanol and, after addition of some graft crystals, stored overnight at -20°C. The precipitate formed was filtered off and dried. A second crop was obtained from the filtrate. Yield 38%, which appeared to be a mixture of several components. A small amount (2.5 g) was chromatographed over two Merck-Fertig C columns in series with hexane/ether 4:1 as the eluent. The eluate obtained was evaporated and the residue was triturated with hexane. Yield 800 mg of a solid which was a

1:1 mixture of the E and Z isomers of compound (a). Rf toluene/ethyl acetate 9:1 = 0.52; toluene = 0.19; hexane/chloroform 1:1 = 0.21 and hexane/ether 4:1 = 0.19. Mp. 52 to 58°C.

PMR spectrum (60 MHz, $CDCl_3$, $\delta$ in ppm): 1.12 and 1.15 (2x t, 3H); 2.07 and 2.32 (2x s, 3H); 2.45 (s, 3H); 2.41 and 2.82 (2x q, 2H); 7.45 (d, 2H); 7.93 (d, 2H).

IR-spectrum (KBr, $cm^{-1}$): 2110; 1598; 1330; 1160.

Further elution of the column resulted in a compound showing an Rf of 0.14. This compound appeared to be compound (b). Yield 800 mg. Rf toluene/ethylacetate = 0.47; toluene = 0.15; hexane/chloroform 1:1 = 0.15. Mp. 82°C.

PMR spectrum (60 MHz, $CDCl_3$, $\delta$ in ppm): 1.72 (d, 3H; 1.80 (m, 3H); 2.47 (s, 3H); 5.00 (s, 1H); 5.76 (q, 1H); 7.45 (d, 2H); 7.90 (d, 2H).

IR spectrum ($CHCl_3$, $cm^{-1}$): 2143; 1665; 1600; 1328; 1157.

Example 28

Preparation of α-(3-cholestanylidene)-α-(4-toluenesulphonyl)-methylisocyanide.

To a mixture of 6.4 mmoles of N-/¯α-(3-cholestanylidene)-4-toluenesulphonylmethyl_7-formamide (cf. Example 60), 32 mmoles of N-methylmorpholine and 35 ml of dimethoxyethane a solution of 0.8 ml $POCl_3$ in 5 ml of dimethoxyethane was added dropwise at -30°C. The mixture was stirred at -10°C for an hour and at 0°C for 90 minutes. The mixture was poured into 150 ml of iced water and extracted with ethyl acetate. The extract was dried and evaporated. The residue was chromatographed over a Merck-Fertig C column with hexane/ether 4:1 as the eluent. The eluate was evaporated and the residue was mixed with diethylether. The ether solution was brought into a crystallisation vessel and the ether was evaporated by means of a stream of nitrogen guided over the mixture. Yield 23%. Rf toluene/ethyl acetate 9:1 = 0.72; Rf chloroform = 0.60; Rf toluene = 0.43; Rf hexane/ether 4:1 = 0.18. Melting point 133.5°C.

PMR spectrum (60 MHz, $CDCl_3$, $\delta$ in ppm): 0.64 (s, 3H); 0.82 (s, 3H); 0.83 (d, 3H); 0.90 (s, 6H); 0.8 to 2.4 (m, 31 H); 2.48 (s, 3H); 7.33 (d, 2H); 7.92 (d, 2H).

IR spectrum (KBr, $cm^{-1}$): 2115; 2100; 1600; 1332; 1160.

Example 29

Preparation of 1-isocyano-2-methyl-1-(4-toluenesulphonyl)-4-(1,1,3-trimethylcyclohex-2-en-2-yl)-3-trans-1,3-butadiene.

To a mixture of 8.3 mmoles of 1-formamido-2-methyl-1-(4-toluenesulphonyl)-4-(1,1,3-trimethylcyclohex-2-en-2-yl)-3-trans-1,3-butadiene (cf. Example 61), 41.5 mmoles of N-methylmorpholine and 35 ml of dimethoxyethane a solution of 1 ml $POCl_3$ in 6.5 ml of dimethoxyethane was added dropwise at -20°C. The mixture was stirred at -20°C for an hour and an additional amount of 1 ml $POCl_3$ was added. After stirring at 0°C for 2.5 hours the mixture was poured into 200 ml of iced water and extracted with methylene chloride. The extract was dried and evaporated. The residue was treated again in the same manner. The mixture was stirred for 15 minutes and recovered. The extract was dried, evaporated to 50 ml and filtered over aluminium oxide (act. II to III, 3x10 cm). The filtrate was evaporated and the residue was mixed with 3 ml of methanol and stored at -20°C. Then the methanol was decanted off and the remaining oil was evaporated by means of an oil pump. A very tacky oil that dit not crystallise was the result. Yield 26%, Rf toluene/ethyl acetate 9:1 = 0.68.

PMR spectrum (60 MHz, $CDCl_3$, $\delta$ in ppm): 1.03 (s, 6H); 1.5 (m, 4H); 2.0 (m, 2H); 1.76 (s, 3H); 2.42 (s, 3H); 2.46 (s, 3H); 6.60 (d, 1H, J = 16 Hz); 6.97 (d, 1H); 7.40 (d, 2H); 7.93 (d, 2H).

IR spectrum (film between NaCl, $cm^{-1}$): 2152; 1595; 1340; 1165; 970.

## Example 30

Preparation of 2-[1-isocyano-1-(4-toluenesulphonyl)-ethen-2-yl]-1-methylpyrrole.

To a mixture of 20 mmoles of 2-[1-formamido-1-(4-toluenesulphonyl)-ethen-2-yl]-1-methylpyrrole (cf. Example 62), 0.1 mole of triethylamine and 60 ml of dimethoxyethane a solution of 2 ml $POCl_3$ in 4 ml of dimethoxyethane was added dropwise at -20°C. The mixture was stirred at -20°C for 15 minutes and at 0°C for an hour and an additional amount of 1 ml of $POCl_3$ was added. After stirring at 0°C for 30 minutes the reaction mixture was poured into 200 ml of iced water. The precipitate formed was filtered off, washed with water and dried. Yield 89%. Melting point 145°C.

PMR spectrum (60 MHz, $CDCl_3$, $\delta$ in ppm): 2.43 (s, 3H); 3.73 (s, 3H); 6.33 (m, 1H), 6.95 (m, 1H); 7.30 (m, 1H); 7.36 (d, 2H); 7.55 (s, 1H), 7.85 (d, 2H).

IR spectrum (KBr, $cm^{-1}$): 2098; 1601; 1488; 1324; 1151.

Example 31

Preparation of 2-(4-phenylphenyl)-1-isocyano-1-(4-toluenesulphonyl)-1-propene.

To a solution of 15 ml of 2-(4-phenylphenyl)-1-formamido-1-(4-toluenesulphonyl)-1-propene (cf. Example 63), 12.5 ml of triethylamine and 45 ml of dimethoxyethane a solution of 1.8 ml of $POCl_3$ in 15 ml of dimethoxyethane was added dropwise at -5°C. The mixture was stirred for 2 hours and an additional amount of 1 ml $POCl_3$ was added. After stirring for another 2 hours the reaction mixture was poured into 500 ml of iced water and extracted with methylene chloride. The extract was treated with activated carbon, dried and evaporated. The residue was crystallised twice from methanol. Then the compound was chromatographed over two Merck-Fertig C columns with toluene as the eluent. The component showing an Rf value (toluene) of 0.22 appeared to be the E isomer of the title compound. Yield 25%. Melting point 77°C.

The residual mixture showing an Rf value of 0.16 and 0.18, which did not separate was obtained. According to PMR at 300 MHz the mixture appeared to be a 1:1 mixture of the Z isomer and 2-(4-phenylphenyl)-1-isocyano-1-(4-toluenesulphonyl)-2-propene. The yield of the mixture was 5%, its melting point 130°C.

PMR spectrum (E isomer, 60 MHz, $CDCl_3$, $\delta$ in ppm): 2.47 (s, 2H); 2.64 (s, 3H); 7.3 to 8.15 (m, 13H).

IR spectrum (KBr, $cm^{-1}$): 2104; 1600; 1340; 1160.

Example 32

Preparation of 1-formamido-2-phenyl-1-(4-toluenesulphonyl)-ethene.

A solution of 14.7 g (75 mmoles) of p-toluenesulphonylmethylisocyanide in 50 ml of dry 1,2-dimethoxyethane was added dropwise to a stirred suspension of potassium t-butylate (12 g, about 100 mmoles) in a nitrogen atmosphere at a temperature between -30 and -10°C. Then 75 mmoles of benzaldehyde in 30 ml of 1,2-dimethoxyethane were added dropwise at a temperature between -40 and -30°C. After stirring 30 minutes at -30°C the solution was added to 500 ml of iced water and acidified with acetic acid. The mixture was extracted with dichloromethane, the extract was washed with water and dried with magnesium sulphate. Then the mixture was concentrated and treated with 100 ml of methanol at -20°C. A yield of 19.7 g (87%)

of the title compound was obtained. Crystallisation from methanol resulted in an analytically pure sample with a melting point of 146 to 152°C.

IR spectrum (nujol, $cm^{-1}$): 3320; 1690; 1650; 1330; 1315; 1160.

PMR spectrum (100 MHz, $CDCl_3$, $\delta$ in ppm): 2.44 (s, 3H); 7.2 to 7.9 (m, 10 H); about 7.5 (broad s, 1H); 8.06 (broad s, 1H).

Analysis calculated for $C_{16}H_{15}NO_3S$ (%): C 63.77; H 5.02; N 4.65; S 10.61; found: C 63.5; H 5.0; N 4.7; S 10.6.

Example 33

Preparation of N-(α-adamantylidene-4-toluenesulphonylmethyl)-formamide.

The above-indicated compound was prepared in the way as indicated in Example 32 from 3.75 g (25 mmoles) of adamantone. Yield 7.33 g (85%). Two crystallisations from ethanol resulted in an analytically pure sample, having a melting point of 134 to 136°C.

IR spectrum (nujol, $cm^{-1}$): 3420; 1710; 1295; 1150.

Analysis calculated for $C_{19}H_{23}NO_3S$ (%): C 66.06; H 6.71; N 4.05; S 9.28; found: C 66.1; H 6.7; N 4.0; S 9.3.

Example 34

Preparation of 1-formamido-2-(4-nitrophenyl)-1-(4-toluenesulphonyl)-ethene.

This compound was prepared in the way as indicated in Example 32 from 3.78 g (25 mmoles) of p-nitrobenzaldehyde. By succion a yield of 6.67 g (77%) of a solid was obtained. Melting point (from ethanol) 210°C (decomp.).

IR spectrum (nujol, $cm^{-1}$): 3250; 1695; 1655; 1535; 1360; 1330; 1160.

Analysis calculated for $C_{16}H_{14}N_2O_5S$ (%): C 55.49; H 4.07; N 8.09; S 9.26; found: C 55.4; H 4.1; N 8.1; S 9.2.

Example 35

Preparation of 3,3-dimethyl-1-formamido-1-(4-toluenesulphonyl)-ethene.

The above-mentioned compound was prepared in the way as described in Example 32 from 2.15 g (25 mmoles) of pivaloylaldehyde. Yield 4.56 g (65%). Melting point 117°C.

PMR spectrum (100 MHz, DMSO-$d_6$, $\delta$ in ppm): 1.10 and 1.13 (2x broad s, together 9H); 2.44 (s, 3H); 6.90 and 7.00 (2x broad s, together 1H); 7.38 (d, 2H); 7.88 (d, 2H); 7.92 (broad s, 1H); 9.6 (broad s, 1H).

## Example 36

Preparation of 1-formamido-2,2-diphenyl-1-(4-toluenesulphonyl)-ethene.

In the way as described in Example 32 the above-mentioned compound was prepared from 4.55 g (25 mmoles) of benzophenone. Yield 4.70 g (50%); melting point 130 to 150°C. An analytically pure sample was obtained by crystallisation from ethanol; melting point 162 to 164°C.

IR spectrum (nujol, $cm^{-1}$): 3300; 1705; 1620; 1330; 1160.

Analysis calculated for $C_{22}H_{19}NO_3S$ (%): C 70.01; H 5.07; N 3.71; S 8.49; found: C 69.6; H 5.1; N 3.5; S 8.4.

## Example 37

Preparation of N-(α-cyclohexylidene-4-toluenesulphonyl-methyl)formamide.

This compound was prepared in the way as described in Example 32 from cyclohexanone in a yield of 80%.

## Example 38

Preparation of 1-formamido-1-(4-toluenesulphonyl)-ethene.

A solution of 19.6 g (100 mmoles) of p-toluenesulphonylmethyl isocyanide in 90 ml of 1.2-dimethoxyethane was added dropwise to a stirred suspension of 16 g (about 140 mmoles) of potassium t-butylate in 100 ml of dry 1.2-dimethoxyethane in a nitrogen atmosphere at -60°C. Then gaseous formaldehyde, obtained by depolymerisation of paraformaldehyde (12 g, about 200°C) was introduced at the same temperature during an hour by taking the formaldehyde gas along with the nitrogen. Acetic acid (8 g) was added and the temperature was increased to 20°C. The mixture was added to water and recovered in the way as described in Example 32. Yield 13.6 g (60%). Melting point 90 to 93°C.

PMR spectrum (60 MHz, $CDCl_3$, $\delta$ in ppm): 2.44 (s, 3H); 6.03 (d, J = 3 Hz, 1H); 6.41 (broad s, 1H); 7.38 (d, 2H); 7.88 (d, 2H); 8.23 (broad s, 1H).

IR spectrum (nujol, $cm^{-1}$): 3400; 1710 (sh); 1695; 1640; 1335; 1180; 1150.

Analysis calculated for $C_{10}H_{11}NO_3S$ (%): C 53.32; H 4.92; N 6.22; S 14.23; found: C 53.3; H 4.9; N 6.1; S 14.1.

## Example 39

Preparation of 1-formamido-2-(2-furyl)-1-(4-toluenesulphonyl)-ethene.

This compound was prepared in the way as described in Example 32 from 50 mmoles of p-toluenesulphonylmethylisocyanide, 50 mmoles of potassium t-butylate and 50 mmoles of furfural in a yield of 8.0 g (55%). Melting point 123°C (from ethanol).

PMR spectrum (60 MHz, $CDCl_3$, $\delta$ in ppm): 2.41 (s, 3H); 6.58 (m, 1H); 6.84 (m, 1H); 7.32 (m, 1H); 7.51 (d, 2H); 7.7 (broad s, 1H); 7.84 (d, 2H); 8.20 (broad s, 1H).

IR spectrum (KBr, $cm^{-1}$): 3220; 3175; 1678; 1652; 1641; 1600; 1315; 1150.

## Example 40

Preparation of 1-formamido-2-(4-chlorophenyl)-1-(4-toluenesulphonyl)-ethene.

This compund was prepared in the way as described in Example 32 from 7.0 g (50 mmoles) of p-chlorobenzaldehyde in a yield of 79%. Melting point (after crystallisation from methanol) 169 to 172°C.

PMR spectrum (60 MHz, DMSO-$d_6$, $\delta$ in ppm): 2.38 (s, 3H); 7.4 - 8.1 (m, 10 H); 10.2 (broad s, 1H).

IR spectrum (KBr, $cm^{-1}$): 3250; 1680; 1640; 1315; 1300; 1160.

## Example 41

Preparation of 2-phenyl-1-formamido-1-(4-toluenesulphonyl)-1-propene.

This compound was prepared in the way as described in Example 55 from freshly distilled acetophenone and p-toluenesulphonyl methylisocyanide. Yield 90%. Melting point 148°C. Rf toluene/ethyl acetate 9:1 = 0.18.

PMR spectrum (60 MHz, $CDCl_3$, $\delta$ in ppm): 2.44 (s, 3H); 2.52 and 2.67 (s, 3H); 7.0 - 8.1 (m, 11H).

IR spectrum (FBr, $cm^{-1}$): 3260; 1690; 1675; 1615; 1597; 1320; 1150.

Example 42

Preparation of 1-formamido-2-methyl-2-(furan-2-yl)-1-(4-toluenesulphonyl)-1-propene.

This compound was prepared in the way as described in Example 55. After recovery the oil obtained was crystallised from methanol. Yield 75%. Rf toluene/ethyl acetate 1:1 = 0.47. Melting point 113°C.

PMR spectrum (60 MHz, $CDCl_3$, $\delta$ in ppm): 2.42 (s, 3H); 2.47 and 2.64 (2x s, 3H); 6.53 (m, 1H); 6.90 (m, 1H); 7.3 to 8.4 (m, 7H) (from the absorptions 2.47 and 2.64 the occurrence of rotamers appears).

IR spectrum (KBr, $cm^{-1}$): 3270; 1680; 1610; 1320; 1310; 1145.

Example 43

Preparation of N-/α-(tetrahydropyran-4-ylidene)-4-toluenesulphonylmethyl/-formamide.

This compound was prepared in the way as described in Example 55. After recovery the oil obtained was crystallised from methanol. Yield 60%. Rf toluene/ethyl acetate 1:1 = 0.25. Melting point 112.5°C.

PMR spectrum (60 MHz, $CDCl_3$, $\delta$ in ppm): 2.43 (s, 3H); 2.35 (m, 2H); 3.08 (m, 2H); 3.78 (m, 4H); 7.3 to 8.0 (m, 6H); 8.23 (s, 1H).

IR spectrum (KBr, $cm^{-1}$): 3280; 1670; 1610; 1320; 1150.

Example 44

Preparation of 4-toluenesulphonyl-5-heptyl-2-oxazoline (a) and 1-formamido-1-(4-toluenesulphonyl)-1-nonene (b).

To a suspension of 5.6 g potassium t-butylate in 100 ml of dimethoxyethane (cooled to -70°C), a solution of 9.75 g (50 mmoles) of p-toluenesulphonylmethylisocyanide in 50 ml of dimethoxyethane was added dropwise, with cooling, within 5 minutes. The potassium t-butylate dissolved. The mixture was stirred for 10 minutes and a solution of 6.4 g (50 mmoles) of octanal in 25 ml of dimethoxyethane was added dropwise at -60°C within 10 minutes. The mixture was stirred at -60°C for half an hour and then cooling was discontinued. At -30°C 2.85 ml of acetic acid were added dropwise. The mixture was evaporated and the residue was mixed with 500 ml of ethyl acetate and

and 250 ml of water. The ethyl acetate phase was dried and evaporated to nearly dryness, and n-hexane was added. The solid was filtered off and dried. Yield 5.5 g (34%) of compound (a). Rf toluene/ethyl acetate 9:1 = 0.36. Melting point 105°C.

By using more potassium t-butylate and starting from a more pure octanal formamide (b) was formed as follows:

To a suspension of 8.0 g (70 mmoles) of potassium t-butylate in 30 ml of dimethoxyethane, cooled to -70°C, a solution of 9.75 g (50 mmoles) of p-toluenesulphonylmethylisocyanide in 30 ml of dimethoxyethane was added dropwise, with cooling, within 5 minutes. The temperature increased to -50°C. Almost all potassium t-butylate dissolved. The mixture was stirred for about 10 minutes and a solution of 6.4 g (50 mmoles) of distilled octanal in 10 ml of dimethoxyethane was added dropwise. By cooling the temperature of the reaction mixture was kept between -40 and -30°C. The mixture was stirred at -30°C for 30 minutes and, with stirring, poured into 500 ml of iced water. Acetic acid (5 ml, 85 mmoles) was added and the mixture was extracted with 200 ml and then with 100 ml of methylene chloride. The methylene chloride phase was washed with 200 ml of water, dried with $MgSO_4$ and evaporated to 15.3 g of an oily residue. The oil was dissolved in 15 ml of methanol and stored overnight at -20°C. The precipitate formed was filtered off and washed. Yield 4.9 g. A second crop weighed 2.6 g, bringing the total yield of compound (b) to 46%. Rf toluene/ethyl acetate 1:1 = 0.53. Melting point 69°C.

PMR spectrum compound (a) (60 MHz, $CDCl_3$, $\delta$ in ppm): 0.9 (t, 3H); 1.1 to 1.9 (m, 12 H); 2.43 (s, 3H); 4.7 to 5.3 (m, 2H); 7.0 (d, 2H); 7.38 (d, 2H); 7.85 (d, 2H).

IR spectrum compound (a) (KBr, $cm^{-1}$): 1660; 1620; 1597; 1316; 1150; 1120; 1087; 670.

PMR spectrum compound (b) (60 MHz, $CDCl_3$, $\delta$ in ppm): 0.9 (1, 3H); 1.3 (broad s, 10 H); 2.2 (m, 2H); 2.42 (s, 3H); 7.05 (t, 1H); 7.3 to 8.1 (m, 6H).

IR spectrum compound (b) (KBr, $cm^{-1}$): 3270; 1700; 1680; 1647; 1597; 1323; 1155.

## Example 45

Preparation of N-/α-(thian-4-ylidene)-4-toluenesulphonylmethyl/-formamide.

This compound was prepared in the way as described in Example 55. After recovery the organic phase was evaporated, yielding 95% of the title compound. Rf toluene/ethyl acetate 1:1 = 0.5. Melting point 126°C.

PMR spectrum (60 MHz, $CDCl_3$, $\delta$ in ppm): 2.42 (s, 3H); 2.5 to 2.9 (m, 6H); 3.2 (m, 2H); 7.3 to 8.0 (m, 5H); 8.26 (s, 1H).

IR spectrum (KBr, $cm^{-1}$): 3250; 1680; 1635; 1600; 1325; 1160.

Example 46

Preparation of 1-formamido-2-methyl-2-(thien-2-yl)-1-(4-toluenesulphonyl)-1-propene.

This compound was prepared in the way as described in Example 55. After pouring the mixture into water and neutralisation the solid formed was filtered off and dried. Yield 96%. Rf toluene/ethyl acetate 1:1 = 0.5. Melting point 110°C.

PMR spectrum (60 MHz, $CDCl_3$, $\delta$ in ppm): 2.46 (s, 3H); 2.60 and 2.81 (2x s, 3H); 7.0 to 8.3 (m, 9H).

IR spectrum (KBr, $cm^{-1}$): 3250; 1697; 1683; 1599; 1498; 1314; 1145.

Example 47

Preparation of N-(α-cycloheptylidene-4-toluenesulphonyl-methyl)-formamide.

This compound was prepared in the way as described in Example 55. Yield 72%. Rf toluene/ethyl acetate 1:1 = 0.55. Melting point 84°C.

PMR spectrum (60 MHz, $CDCl_3$, $\delta$ in ppm): 1.51 (broad s, 8H); 2.37 (broad s, 5H); 2.87 (m, 2H); 7.2 to 7.96 (m, 5H); 8.16 (d, 1H).

IR spectrum (KBr, $cm^{-1}$): 3270; 1685; 1668; 1612; 1597; 1320; 1145.

Example 48

Preparation of 1-formamido-2-(2-thienyl)-1-(4-toluenesulphonyl)-ethene.

The preparation of this compound was as described in Example 55. After pouring the mixture into water and neutralisation with acetic acid a solid was formed. That solid was filtered off and dried in a desiccator connected to a vacuum pump during two days.

Yield 94%. Rf toluene/ethyl acetate = 0.7.

PMR spectrum (60 MHz, $CDCl_3$, δ in ppm): 2.42 (s, 3H); 7.1 to 8.3 (m, 10 H).

IR spectrum (KBr, $cm^{-1}$): 3125; 1698; 1686; 1630; 1598; 1318; 1147.

Example 49

Preparation of N-[α-(1-methylpiperid-4-ylidene)-4-toluene sulphonylmethyl]-formamide.

This compound was prepared as described in Example 55. After pouring the mixture into water and neutralisation with acetic acid it was extracted three times with 150 ml of methylene chloride. This resulted, after recovery, in 3.3 g. The aqueous phase was made alkaline with sodium carbonate and was extracted with 500 ml of methylene chloride, resulting, after recovery, in another 5.0 g. Total yield 54%. Rf methanol = 0.4. Melting point 165°C.

PMR spectrum (60 MHz, $CDCl_3$, δ in ppm): 2.28 (s, 3H); 2.4 (m, 6H); 2.43 (s, 3H); 3.1 (m, 2H); 7.3 to 8.25 (m, 6H).

IR spectrum (KBr, $cm^{-1}$): 3145; 2750; 1688; 1638; 1597; 1316; 1300; 1148.

Example 50

Preparation of 2-phenyl-1-formamido-1-(4-toluenesulphonyl)-1-hexene.

To a suspension of 4 g of potassium t-butoxide in 13 ml of dry dimethoxyethane a solution of 0.025 mole p-toluenesulphonyl-methylethane in 18 ml of dimethoxyethane was added dropwise at -10 to -30°C. Then 0.025 mole of valerophenone was added at -35°C. The reaction mixture was stirred at -30°C for 30 minutes and poured into 175 ml of iced water acidified with 2.3 ml of glacial acetic acid. The mixture was extracted with methylene chloride and the extract was dried and evaporated to 7.6 g of an oil. The oil was chromatographed over silicagel with toluene/ethyl acetate 6:1 as the eluent. The oil obtained was triturated with ethyl acetate and solidified. Yield 42%. Rf toluene/ethyl acetate 1:1 = 0.2. Melting point 124°C.

PMR spectrum (60 MHz, $CDCl_3$, δ in ppm): 1.28 and 1.34 (t, 3H); 1.77 (m, 4H); 2.40 (s, 3H); 2.97 (m, 2H); 7.0 to 7.5 (m, 9H); 7.6 to 8.1 (m, 2H).

IR spectrum (KBr, $cm^{-1}$): 3160; 3080; 1690; 1585; 1315;

1155.

Example 51

Preparation of N-(α-(cyclooctylidene-4-toluenesulphonyl-methyl)-formamide.

To a suspension of 8 g of potassium t-butylate in 25 ml of dimethoxyethane a solution of 0.05 mole of p-toluenesulphonylmethylisocyanide in 35 ml of dimethoxyethane was added dropwise at -20°C, followed by the addition of 0.05 mole of freshly distilled cyclo-octanone in 20 ml of dimethoxyethane at -35°C. The mixture was stirred at -30°C for 30 minutes and poured into 350 ml of iced water acidified with 4.6 ml of glacial acetic acid. The mixture was extracted with methylene chloride and the extract was dried and evaporated to 16.9 g of an oil which solidified very slowly. Rf toluene/ethyl acetate 1:1 = 0.73. Melting point 70 to 73°C.

PMR spectrum (60 MHz, $CDCl_3$, $\delta$ in ppm): 1.45 to 2.1 (m, 10 H); 2.2 to 3.2 (m, 4H); 2.42 (s, 3H); 7.1 to 8.2 (m, 6H).

IR spectrum (KBr, $cm^{-1}$): 3250; 1710; 1695; 1605; 1320; 1155.

Example 52

Preparation of 1-formamido-2-methyl-1-(4-toluenesulphonyl)-1-undecene.

To a suspension of 8 g of potassium t-butylate in 26 ml dimethoxyethane a solution of 0.05 mole of p-toluenesulphonylmethylisocyanide in 36 ml of dimethoxyethane was added dropwise at -20°C, followed by a solution of 0.05 mole of 2-undecanone in 20 ml of dimethoxyethane at -30°C. The mixture was stirred at -30°C for 30 minutes and poured into 350 ml of iced water acidified with 4.6 ml of glacial acetic acid. The mixture was extracted with methylene chloride and the extract was dried, treated with activated carbon and evaporated. The residue appeared to consist of the E and Z isomers, which were separated by chromatography over silicagel (90 x 4.5 cm) with toluene/ethyl acetate 3:1 = 0.5 and melting point 77°C, and 16% of the Z isomer, Rf toluene/ethyl acetate 3:1 = 0.3, melting point 78.5°C.

PMR spectrum E isomer (60 MHz, $CDCl_3$, $\delta$ in ppm): 0.88 (t, 3H); 1.20 (m, 14 H); 2.13 (t, 2H); 2.20 and 2.34 (2x s, 3H); 2.42 (s, 3H); 7.1 to 8.5 (m, 6H).

IR spectrum E isomer (KBr, $cm^{-1}$): 3310; 1685; 1630; 1595; 1312; 1299; 1142.

PMR spectrum Z isomer (60 MHz, $CDCl_3$, $\delta$ in ppm): 0.90 (t, 3H); 1.24 (m, 14 H); 1.82 and 1.93 (2x s, 3H); 2.40 (s, 3H); 2.64 (t, 2H); 7.1 to 8.1 (m, 6H).

IR spectrum Z isomer (KBr, $cm^{-1}$): 3240; 1665; 1625; 1590; 1320; 1150.

Example 53

Preparation of 1-formamido-2-(4-nitrophenyl)-1-(4-toluenesulphonyl)-1-propene.

To a suspension of 8 g of potassium t-butylate in 26 ml of dimethoxyethane a solution of 50 mmoles of p-toluenesulphonylmethylisocyanide in 36 ml of dimethoxyethane was added dropwise at -20°C, followed by the addition of a solution of 50 mmoles of 4-nitroacetophenone in 20 ml of dimethoxyethane at -45°C. The reaction mixture was stirred for 30 minutes and poured into 350 ml of iced water acidified with 4.6 ml of glacial acetic acid. The mixture was extracted with methylene chloride and the extract was washed several times with iced water, dried, treated with activated carbon and evaporated. The residue was chromatographed over silicagel (90 x 4.5 cm) with toluene/ethyl acetate 3:1 as the eluent. The fractions of Rf = 0.33 were collected and evaporated. The residue was triturated with 50 ml of methanol. Yield 68%, melting point 194°C.

PMR spectrum (60 MHz, $CDCl_3$/DMSO, $\delta$ in ppm): 2.44 (s, 3H); 2.44 and 2.61 (2x s, 3H); 7.2 to 8.3 (m, 10 H).

IR spectrum (KBr, $cm^{-1}$): 3250; 1695; 1665; 1620; 1587; 1510; 1340; 1317; 1145.

Example 54

Preparation of 1-formamido-2-(4-methoxyphenyl)-1-(4-toluenesulphonyl)-ethene.

To a suspension of 8 g of potassium t-butylate in 26 ml of dimethoxyethane a solution of 50 mmoles of p-toluenesulphonylmethylisocyanide in 36 ml of dimethoxyethane was added dropwise at -20°C, followed by the addition of a solution of 50 mmoles of 4-methoxybenzaldehyde in 20 ml of dimethoxyethane. The reaction mixture was stirred for 45 minutes and poured into 350 ml of iced water, acidified with 4.6 ml of glacial acetic acid. The mixture was extracted with methylene chloride and the extract was dried and evaporated. Yield 92%. Rf toluene/ethyl acetate 3:1 = 0.12. Melting point 160 to 164°C.

PMR spectrum (60 MHz, $CDCl_3$, $\delta$ in ppm): 2.40 (s, 3H); 3.79 (s, 3H); 6.81 and 6.95 (s, 1H); 7.1 to 8.1 (m, 10 H).

IR spectrum (KBr, $cm^{-1}$): 3150; 1695; 1680; 1632; 1502; 1310; 1265; 1155.

Example 55

Preparation of 1-formamido-1-(4-toluenesulphonyl)-1-pentene.

To a suspension of 16 g of potassium t-butylate in 50 ml of dimethoxyethane a solution of 0.1 mole of p-toluenesulphonylmethylisocyanide in 70 ml of dimethoxyethane were added dropwise at -70°C, followed by the addition of 0.1 mole of freshly distilled butanal in 40 ml of dimethoxyethane at -40°C. The mixture was stirred at -20°C for an hour and was poured into 700 ml of iced water, acidified with 9.2 ml of glacial acetic acid. The mixture was extracted with methylene chloride and the extract was dried and evaporated. The residue was triturated with methanol at 0°C and filtered off. A second crop was obtained from the filtrate. Total yield 56%. Rf toluene/ethyl acetate 1:1 = 0.34. Mp. 100°C.

PMR spectrum (60 MHz, $CDCl_3$, $\delta$ in ppm): 0.96 (t, 3H); 1.54 (m, 2H); 2.16 (q, 2H); 2.40 (s, 3H); 7.01 and 7.08 (t, 1H); 7.35 (d, 2H); 7.80 (m, 3H); 8.11 (s, 1H).

IR spectrum (KBr, $cm^{-1}$): 3250; 1685; 1655; 1595; 1325; 1165.

Example 56

Preparation of N-(α-cyclohexylidenebenzenesulphonylmethyl)-formamide.

To a suspension of 8 g of potassium t-butylate in 25 ml of dimethoxyethane a solution of 50 mmoles of phenylsulphonylmethylisocyanide in 35 ml of dimethoxyethane was added dropwise at -20°C, followed by the addition of 50 mmoles of cyclohexanone in 20 ml of dimethoxyethane at -35°C. The reaction mixture was stirred for an hour and poured into 350 ml of iced water acidified with 4.6 ml of glacial acetic acid. The mixture was extracted with methylene chloride and the extract was treated with activated carbon, dried and evaporated. The residue was mixed with 15 ml of methanol, grafted and stored at -20°C for a week. The precipitate formed was filtered off and washed with some methanol at -20°C. Yield 30%. Rf toluene/ethyl acetate 9:1 = 0.07. Melting point 90°C.

PMR spectrum (60 MHz, $CDCl_3$, $\delta$ in ppm): 1.62 (m, 6H);

2.25 (m, 2H); 2.9 (m, 2H); 7.5 to 8.3 (m, 7H).

IR spectrum (KBr, $cm^{-1}$): 3270; 1680; 1620; 1385; 1375; 1130.

Example 57

Preparation of 1-formamido-2-pentyl-1-(4-toluenesulphonyl)-1-heptene.

To a suspension of 8 g of potassium t-butoxide in 26 ml of dimethoxyethane a solution of 50 mmoles of p-toluenesulphonylmethylisocyanide in 36 ml of dimethoxyethane was added dropwise at -20°C, followed by the addition of 50 mmoles of 6-undecanone in 20 ml of dimethoxyethane. After stirring for 30 minutes the mixture was poured into 350 ml of water acidified with 4.6 ml of glacial acetic acid. The mixture was extracted with methylene chloride and the extract was treated with activated carbon, dried and evaporated. Yield 88%. Rf toluene/ethyl acetate 3:1 = 0.5. Melting point 60°C.

PMR spectrum (60 MHz, $CDCl_3$, $\delta$ in ppm): 0.85 (t, 6H); 1.26 (m, 12 H); 2.15 (m, 2H); 2.40 (s, 3H); 2.65 (m, 2H); 6.9 to 8.1 (m, 6H).

IR spectrum (KBr, $cm^{-1}$): 3300; 1680; 1625; 1595; 1320; 1310; 1150.

Example 58

Preparation of 2-(5-chloro-2-thienyl)-1-formamido-1-(4-toluenesulphonyl)-1-propene.

To a solution of 59 g of 2-chlorothiophene in 102 g of acetic acid anhydride 1.75 ml of perchloroacetic acid were added. The mixture was stirred at room temperature for 24 hours and, after cooling the mixture to 5°C, 375 ml of 4N sodium hydroxide were added dropwise. The hydrolysate was extracted with diethyl ether and the extract was washed with an aqueous solution of 10% sodium bicarbonate, dried, evaporated and distilled <u>in vacuo</u>. Yield 67% of 2-acetyl-5-chlorothiophene having a boiling point of 68°C at 0.7 mm Hg, a melting point of 40 to 45°C and an Rf value (toluene/ethyl acetate 6:1) of 0.44.

To a suspension of 8 g of potassium t-butoxide in 25 ml of dimethoxyethane a solution of 50 mmoles of p-toluenesulphonylmethylisocyanide in 35 ml of dimethoxyethane was added dropwise at -70°C, followed by the addition of a solution of 50 mmoles of 5-chlorothiophene in 20 ml of dimethoxyethane at -40°C. The mixture was stirred at -30°C for an hour and was poured into 350 ml of iced

water, acidified with 4.6 ml of glacial acetic acid. The mixture was extracted with methylene chloride and the extract was treated with activated carbon, dried and evaporated. Yield 99%. Rf toluene/ethyl acetate 6:1 = 0.23. Melting point 161 to 162°C.

PMR spectrum (60 MHz, DMSO, $\delta$ in ppm): 2.38 (s, 6H); 7.15 to 8.4 (m, 7H); 10.24 (broad s, 1H).

IR spectrum (KBr, $cm^{-1}$): 3100; 1700; 1655; 1590; 1315; 1140.

Example 59

Preparation of 1-formamide-2-(4-pyridyl)-1-(4-toluene-sulphonyl)-ethene.

To a suspension of 8 g of potassium t-butoxide in 25 ml of dimethoxyethane a solution of 50 mmoles of p-toluenesulphonylmethylisocyanide in 35 ml of dimethoxyethane was added dropwise at -70°C, followed by the addition of 50 mmoles of pyridine-4-aldehyde in 20 ml of dimethoxyethane at -40°C. The reaction mixture was stirred for an hour and poured into 350 ml of iced water acidified with acetic acid. The mixture was extracted with methylene chloride and the aqueous phase was brought to pH 7 and extracted again with methylene chloride. The collected extract was treated with activated carbon, dried and evaporated. Yield 80%. Rf ethyl acetate = 0.21. Melting point 155 to 157°C.

PMR spectrum (60 MHz, DMSO, $\delta$ in ppm): 2.36 (s, 3H); 7.3 to 7.9 (m, 8H); 8.55 (d, 2H); 10.35 (broad s, 1H).

IR spectrum (KBr, $cm^{-1}$): 3320; 3100; 1695; 1640; 1590; 1300; 1145.

Example 60

Preparation of N-[α-(3-cholestanylidene)-4-toluenesulpho-nylmethyl]-formamide.

To a suspension of 2.1 g of potassium t-butoxide in 10 ml of dimethoxyethane a solution of 12.9 mmoles of p-toluenesulphonylmethylisocyanide in 15 ml of dimethoxyethane was added dropwise at -60°C, followed by the addition of a solution of 12.9 mmoles of 3-cholestanone in 60 ml of dimethoxyethane at -35°C. After stirring for 15 minutes the reaction mixture was poured into 150 ml of iced water acidified with 1.3 ml of glacial acetic acid. The precipitate formed was filtered off, washed with water and dried. Yield 97%. Rf toluene/ethyl acetate 9:1 = 0.21. Melting point 122°C.

PMR spectrum (60 MHz, $CDCl_3$, $\delta$ in ppm): 0.64 (s, 3H);

0.82 (s, 3H); 0.84 (d, 3H); 0.90 (s, 6H); 0.8 to 2.4 (m, 31 H); 2.43 (s, 3H); 7.3 to 8.3 (m, 6H).

IR spectrum (KBr, $cm^{-1}$): 3300; 1710; 1700; 1685; 1620; 1325; 1310; 1155.

## Example 61

Preparation of 3-acetyl-4-(1,1,3-trimethylcyclohex-2-en-2-yl)-pyrrole (a) and 1-formamido-2-methyl-1-(4-toluenesulphonyl)-4-(1,1,3-trimethylcyclohex-2-en-2-yl)-3-trans-1,3-butadiene (b).

To a suspension of 8 g of potassium t-butoxide in 40 ml of dimethoxyethane a solution of 50 mmoles of p-toluenesulphonylmethylisocyanide in 50 ml of dimethoxyethane was added dropwise at -30°C, followed by the addition of 50 mmoles of β-ionone in 30 ml of dimethoxyethane at -35°C. After stirring for half an hour the reaction mixture was poured into 750 ml of iced water acidified with 5 ml of glacial acetic acid. The mixture was extracted twice with methylene chloride and twice with ethyl acetate. The combined extract was dried and evaporated. The residue was stirred with methanol. The remaining solid was filtered off and dried and the filtrate was stored at -20°C resulting in a second crop. The solid was compound (a). The mother liquor was chromatographed over silicagel (90x4 cm) with toluene/ethyl acetate 9:1 as the eluent. The fractions with the component showing an Rf of 0.22 were collected and evaporated. The residue was an oil and appeared to be compound (b). Yield 16%. Further elution resulted in another amount of compound (a) at Rf 0.15. Yield of compound (a) 48%, melting point 188°C.

PMR spectrum compound (a) (60 MHz, DMSO-$d_6$, $\delta$ in ppm): 0.74 (s, 3H); 0.96 (s, 3H); 1.28 (s, 3H); 1.6 (m, 4H); 2.0 (m, 2H); 2.24 (s, 3H); 6.4 (double d, 1H); 7.52 (double d, 1H); 11.4 (s, 1H).

$^{13}$CMR-spectrum (DMSO-$d_6$, $\delta$ in ppm): 20.55; 22.86; 29.12; 29.52; 30.15; 33.15; 35.82; 40.34; 119.49; 126.03; 129.36; 130.74; 134.64; 193.77.

IR spectrum (KBr, $cm^{-1}$): 3285; 1648; 1620; 1520; 1390; 1360; 1170.

PMR spectrum compound (b) (60 MHz, $CDCl_3$, $\delta$ in ppm): 0.98 (s, 6H); 1.5 (m, 4H); 1.63 (s, 3H); 2.0 (m, 2H); 2.31 (s, 3H); 2.40 and 2.47 (s, 3H); 6.0 to 7.1 (m, 2H); 7.1 to 8.3 (m, 6H).

IR spectrum (film between NaCl, $cm^{-1}$): 3300; 1715; 1700; 1605; 1330; 1310; 1160; 975.

Example 62

Preparation of 2-[1-formamido-1-(4-toluenesulphonyl)-ethen-2-yl]-1-methylpyrrole.

To a suspension of 8 g of potassium t-butoxide in 40 ml of dimethoxyethane a solution of 50 mmoles of p-toluenesulphonylmethylisocyanide in 50 ml of dimethoxyethane was added dropwise at -30°C, followed by the addition of a solution of 50 mmoles of N-methylpyrrole-2-aldehyde in 20 ml of dimethoxyethane. After stirring for 30 minutes the reaction mixture was poured into iced water acidified with 5 ml of glacial acetic acid. The solid formed was filtered off, washed with water and dried. Yield 63%. Rf toluene/ethyl acetate 1:1 = 0.47. Melting point 167°C.

PMR spectrum (60 MHz, DMSO, $\delta$ in ppm): 2.38 (s, 3H); 3.75 (s, 3H); 6.17 (m, 1H); 6.60 (m, 1H); 7.10 (m, 1H); 7.3 (d, 2H); 7.9 (d, 2H); 7.71 (s, 1H); 8.0 (s, 1H); 10.0 (s, 1H).

IR spectrum (KBr, $cm^{-1}$): 3145; 1700; 1628; 1599; 1318; 1150.

Example 63

Preparation of 2-(4-phenylphenyl)-1-formamido-1-(4-toluenesulphonyl)-1-propene.

To a suspension of 8 g of potassium t-butylate in 25 ml of dimethoxyethane a solution of 50 mmoles of p-toluenesulphonylmethylisocyanide in 35 ml of dimethoxyethane was added dropwise at -20°C, followed by the addition of 50 mmoles of 4-acetylbiphenyl in 40 ml of dimethoxyethane at -35°C. After stirring for half an hour the reaction mixture was poured into 350 ml of iced water acidified with 4.6 ml of glacial acetic acid. The mixture was extracted with methylene chloride and the extract was dried and evaporated. The residue was mixed with 70 ml of methanol and stored overnight at -20°C. The precipitate was filtered off and dried. Yield 45%. Melting point 151°C. Rf toluene/ethyl acetate 9:1 = 0.18. It is appreciated to mention that, if the ketone is added in portions instead of as a solution, 1-biphenyl-1-isocyanoethane is, inter alia, formed as a by-product.

PMR spectrum (60 MHz, DMSO, $\delta$ in ppm): 2.44 (s, 3H); 2.41 and 2.58 (2x s, 3H); 7.35 to 8.1 (m, 14 H); 9.8 (s, 1H).

IR spectrum (KBr, $cm^{-1}$): 3250; 1720; 1695; 1610; 1335; 1160.

Example 64

Preparation of 4-/1-formamido-1-(4-toluenesulphonyl)-ethen-2-yl/-2-phenyl-1,2,3-triazole.

To a suspension of 5.6 g potassium t-butoxide in 40 ml of dimethoxyethane a solution of 35 mmoles of p-toluenesulphonylmethylisocyanide in 40 ml of dimethoxyethane was added dropwise at -70°C, followed by the addition of a solution of 35 mmoles of 2-phenyl-4-formyl-1,2,3-triazole (prepared according to the method described by J.L. Riebsomer and G. Sumrell in J. Org. Chem. 13 (1948) page 807) in 40 ml of dimethoxyethane. In about 2 hours the temperature of the reaction mixture was allowed to rise to about -40°C whereafter the reaction mixture was poured into 500 ml of iced water, acidified with 5 ml of glacial acetic acid. The mixture was extracted with methylene chloride and the extract was dried and evaporated. The residue was crystallised from a mixture of 220 ml of acetone and 600 ml of hexane. Yield 75%. Rf toluene/ethyl acetate 1:1 = 0.55. Melting point 173°C.

PMR spectrum (60 MHz, $CDCl_3$/DMSO-$d_6$, $\delta$ in ppm): 2.42 (s, 3H); 7.3 to 8.3 (m, 12 H); 10.15 (broad s, 1H).

IR spectrum (KBr, $cm^{-1}$): 3260; 1690; 1645; 1590; 1320; 1180; 1140.

Example 65

Ointments are prepared from the compounds according to any one of the Examples 1 to 31 by adding 0.1 to 10 parts by weight of the said compounds to a mixture of 95 parts by weight of paraffine oil and 5 parts by weight of polyethylene, and thoroughly mixing. The ointments are suitable for local administration.

Example 66

Tablets are prepared from the compounds according to any one of the Examples 1 to 31 by thoroughly mixing 125 to 500 mg of the said compounds, 100 to 300 mg of amylum maidis, 5 to 30 mg of polyvinylpyrrolidone, 1 to 20 mg of magnesium stearate and an amount of lactose sufficient for the preparation of a tablet, and forming tablets from the mixtures in the usual way. The tablets are suitable for, e.g. vaginal administration.

Example 67

Dispersible powders are prepared from the compounds according to any one of the Examples 1 to 31 by adding 10 parts by weight of the above-indicated compounds to 86 parts by weight of

bentonite, 2 parts by weight of sodium laurylbenzenesulphonate and 2 parts by weight of sorbitan monolaureate polyethyleneoxide and mixing thoroughly in a suitable mill. The dispersible powders are suitable for the preparation of submersion baths for disinfection of bulbs and the like.

Claims (for all designated countries, except Austria).

1. Isocyanoalkenes having the general formula:

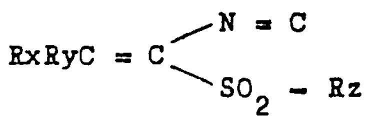 (I) or 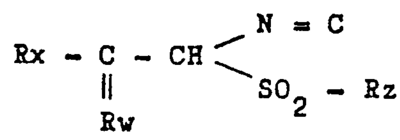 (II)

wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five or six-membered heterocyclic group bearing one or more oxygen, sulphur or nitrogen atoms as hetero atoms, substituted or not substituted by one or more lower alkyl, aryl or halogen groups or Rx and Ry, together with the carbon atom to which they are attached, represent a five- to eight-membered cycloalkylidene group which may bear one or more oxygen, sulphur or nitrogen atoms as hetero atoms, or an adamantylidene or cholestanylidene group, substituted or not substituted by one or more lower alkyl groups, Rw is a lower alkylidene group or Rx and Rw, together with the carbon atom to which they are attached, represent a five- to eight-membered cycloalkenyl group which may bear one or more oxygen, sulphur or nitrogen atoms as hetero atoms, substituted or not substituted by one or more lower alkyl groups, and Rz is an aryl group substituted or not substituted by one or more lower alkyl groups.

2. Isocyanoalkenes according to claim 1, having formula (I), wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five- or six-membered heterocyclic group bearing one or more oxygen, sulphur or nitrogen atoms as the hetero atoms, substituted or not substituted by one or more lower alkyl, aryl or halogen groups, or Rx and Ry, together with the carbon atom to which they are attached, represent a five- to eight-membered cycloalkylidene group which may bear one or more oxygen, sulphur or nitrogen atoms as hetero atoms, or an adamantylidene or cholestanylidene group, substituted or not substituted by one or more lower alkyl groups and Rz is an aryl group substituted or not substituted by one or more lower alkyl groups.

3. Isocyanoalkenes according to claim 2, wherein Rx is an aryl or cyclopentyl group, which may bear an oxygen, sulphur or nitrogen atom as a hetero-atom, and may be substituted by a lower alkyl, nitro, lower alkoxy or halogen group, Ry is a hydrogen atom or a lower alkyl group and Rz is an aryl group substituted or not substituted by one or more lower alkyl groups.

4. Isocyanoalkenes according to claim 3, wherein Rx is a 2-furyl group, Ry is a hydrogen atom or a lower alkyl group and Rz is a tolyl group.

5. 1-Isocyano-2-phenyl-1-(4-toluenesulphonyl)ethene,
α-adamantylidene-α-(4-toluenesulphonyl)methylisocyanide,
1-isocyano-2-(4-nitrophenyl)-1-(4-toluenesulphonyl)ethene,
3,3-dimethyl-1-isocyano-1-(4-toluenesulphonyl)-1-butene,
1-isocyano-2-methyl-1-(4-toluenesulphonyl)-1-propene,
2,2-diphenyl-1-isocyano-1-(4-toluenesulphonyl)ethene,
α-cyclohexylidene-α-(4-toluenesulphonyl)-methylisocyanide,
1-isocyano-1-(4-toluenesulphonyl)-ethene,
α-cyclopentylidene-α-(4-toluenesulphonyl)-methylisocyanide,
2-(2-furanyl)-1-isocyano-1-(4-toluenesulphonyl)ethene,
1-isocyano-2-(4-chlorophenyl)-1-(4-toluenesulphonyl)-ethene,
2-phenyl-1-isocyano-1-(4-toluenesulphonyl)-1-propene,
1-isocyano-2-(2-furanyl)-1-(4-toluenesulphonyl)-1-propene,
α-(tetrahydropyran-4-ylidene)-α-(4-toluenesulphonyl)-methylisocyanide,
α-(2,3-dihydro-6H-pyran-4-yl)-α-(toluenesulphonyl)-methylisocyanide,
1-isocyano-1-(4-toluenesulphonyl)-1-nonene,
α-(thian-4-ylidene)-α-(4-toluenesulphonyl)-methylisocyanide,
α-(3,4-dehydrothian-4-yl)-α-(4-toluenesulphonyl)-methylisocyanide,
1-isocyano-1-(4-toluenesulphonyl)-2-(2-thienyl)-1-propene,
α-cycloheptylidene-α-(4-toluenesulphonyl)-methylisocyanide,
α-(cyclohepten-1-yl)-α-(4-toluenesulphonyl)-methylisocyanide,
2-phenyl-1-isocyano-1-(4-toluenesulphonyl)-1-hexene,
α-(cycloocten-1-yl)-α-(4-toluenesulphonyl)-methylisocyanide,
1-isocyano-2-methyl-1-(4-toluenesulphonyl)-1-undecene,
1-isocyano-2-(4-nitrophenyl)-1-(4-toluenesulphonyl)-1-propene,
1-isocyano-2-(4-nitrophenyl)-1-(4-toluenesulphonyl)-2-propene,
1-isocyano-2-(4-methoxyphenyl)-1-(4-toluenesulphonyl)-ethene,
1-isocyano-1-(4-toluenesulphonyl)-1-pentene,
α-cyclohexylidene-α-benzenesulphonylmethyl-isocyanide,
1-isocyano-2-pentyl-1-(4-toluenesulphonyl)-1-heptene,

1-isocyano-2-pentyl-1-(4-toluenesulphonyl)-1-heptene,
1-isocyano-2-methyl-1-(4-toluenesulphonyl)-1-butene,
1-isocyano-2-methyl-1-(4-toluenesulphonyl)-2-butene,
α-(3-cholestanylidene)-α-(4-toluenesulphonyl)-methylisocyanide,
1-isocyano-2-methyl-1-(4-toluenesulphonyl)-4-(1,1,3-trimethylcyclohex-2-en-2-yl)-3-trans-1,3-butadiene,
2-[1-isocyano-1-(4-toluenesulphonyl)-ethen-2-yl]-1-methylpyrrole and
2-(4-phenylphenyl)-1-isocyano-1-(4-toluenesulphonyl)-1-propene.

6. Process for the preparation of the compounds according to any one of the preceding claims, characterized in that a compound having the general formula:

$$RxRyC = C\begin{matrix} \diagup NH - CHO \\ \diagdown SO_2 - Rz \end{matrix} \qquad (III)$$

wherein Rx, Ry and Rz are as indicated above, with a dehydrating agent.

7. Process according to claim 6, wherein the dehydrating agent is phosphorusoxychloride, preferably in the presence of an acid-binding agent.

8. Process according to claim 7, wherein the reaction is carried out at lower temperatures, e.g. between -50 and +25°C, preferably between -30 and -5°C.

9. Process according to claim 7 or 8, wherein an inert solvent is used, e.g. di-, tri- or tetrachloromethane, ethyl acetate, dioxane, benzene, toluene, xylene, o-dichlorobenzene, acetone, 1,2-dimethoxyethane, bis-(2-methoxyethyl)-ether, dimethylformamide or 1,2-dichloromethane or mixtures thereof.

10. Process according to any one of claims 6 to 9, wherein that preparation is combined with the preparation of the starting formamide from a compound having the formula

$$Rz - SO_2 - CH_2 - N = C \qquad (IV)$$

wherein Rz is as indicated above, with a compound having the formula Rx - CO - Ry, wherein Rx and Ry are as indicated above, to a "one pot process".

11. Formamides having the general formula

$$RxRyC = C\begin{matrix} \diagup NHCHO \\ \diagdown SO_2 - Rz \end{matrix} \qquad (V)$$

wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five- or six-membered heterocyclic group bearing one or more oxygen, sulphur or nitrogen atoms as hetero atoms, substituted or not substituted by one or more lower alkyl, aryl or halogen groups, with the provision that, if Ry is a hydrogen atom, Rx is not a phenyl or t-butyl group, or Rx and Ry together with the carbon atom to which they are attached, represent a five- to eight-membered cycloalkylidene group which may bear one or more oxygen, sulphur or nitrogen atoms as hetero atoms, with the exception of a cyclohexylidene group, or represent an adamantylidene or cholestanylidene group, substituted or not substituted by one or more lower alkyl groups, and Rz is an aryl group substituted or not substituted by one or more lower alkyl groups.

12. Formamides according to claim 11, wherein Rx is an aryl or five-membered heteroaryl group, which may bear an oxygen, sulphur, or nitrogen atom as the heterocyclic atom, and may be substituted by a lower alkyl, nitro, lower alkoxy or halogen group, Ry is a hydrogen atom, or a lower alyl group, with the provision that, if Ry is a hydrogen atom, Rx is not a phenyl group, and Rz is an aryl group substituted or not substituted by one or more lower alkyl groups.

13. Formamides according to claim 12, wherein Rx is a 2-furyl group, Ry is a hydrogen atom or a lower alkyl group and Rz is a tolyl group.

14. N-(α-adamantylidene-4-toluenesulphonylmethyl)-formamide,
1-formamido-2-(4-nitrophenyl)-1-(4-toluenesulphonyl)-ethene,
1-formamido-2,2-diphenyl-1-(4-toluenesulphonyl)-ethene,
1-formamido-1-(4-toluenesulphonyl)-ethene,
1-formamido-2-(2-furanyl)-1-(4-toluenesulphonyl)-ethene,
1-formamido-2-(4-chlorophenyl)-1-(4-toluenesulphonyl)-ethene,
1-formamido-2-phenyl-1-(4-toluenesulphonyl)-1-propene,
1-formamido-2-(2-furanyl)-1-(4-toluenesulphonyl)-1-propene,
N-tetrahydropyran-4-ylidene-(4-toluenesulphonyl)-methylformamide,
1-formamido-1-(4-toluenesulphonyl)-1-nonene,
N-[α-(thian-4-ylidene)-4-toluenesulphonylmethyl]-formamide,
1-formamido-2-methyl-2-(thien-2-yl)-1-(4-toluenesulphonyl)-1-propene,

N-(α-cycloheptylidene-4-toluenesulphonylmethyl)-formamide,
1-formamido-2-(2-thienyl)-1-(4-toluenesulphonyl)-ethene,
N-[α-(1-methylpiperid-4-ylidene)-4-toluenesulphonylmethyl]formamide,
2-phenyl-1-formamido-1-(4-toluenesulphonyl)-1-hexene,
N-(α-cyclooctylidene-4-toluenesulphonylmethyl)-formamide,
1-formamido-2-methyl-1-(4-toluenesulphonyl)-1-undecene,
1-formamido-2-(4-nitrophenyl)-1-(4-toluenesulphonyl)-1-propene,
1-formamido-2-(4-methoxyphenyl)-1-(4-toluenesulphonyl)-ethene,
2-formamido-1-(4-toluenesulphonyl)-1-pentene,
N-(α-cyclohexylidenebenzenesulphonylmethyl)-formamide,
1-formamido-2-pentyl-1-(4-toluenesulphonyl)-1-heptene,
2-(5-chloro-2-thienyl)-1-formamido-1-(4-toluenesulphonyl)-1-propene,
1-formamido-2-(4-pyridyl)-1-(4-toluenesulphonyl)-ethene,
N-[α-(3-cholestanylidene)-4-toluenesulphonylmethyl]-formamide,
1-formamido-2-methyl-1-(4-toluenesulphonyl)-4-(1,1,3-trimethylcyclohex-2-en-2-yl)-3-trans-1,3-butadiene,
2-[1-formamido-1-(4-toluenesulphonyl)-ethen-2-yl]-methylpyrrole,
2-(4-phenylphenyl)-1-formamido-1-(4-toluenesulphonyl)-1-propene.

15. Pharmaceutical or fungicidal preparation in which the active component is present together with the usual formulation agents, wherein the active component is selected from one or more of the compounds according to any one of claims 1 to 5 or prepared according to the process according to any one of claims 6 to 10.

16. Process for the treatment of plant diseases, especially for disinfecting bulbs and tubers, against mould growth, wherein a preparation according to claim 15 is used.

Claims for Austria

1. Process for the preparation of isocyanoalkenes having the general formula:

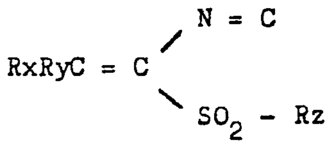 (I) or 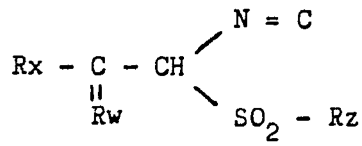 (II)

wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five- or six-membered heterocyclic group bearing one or more oxygen, sulphur or nitrogen atoms substituted or not substituted by one or more lower alkyl, aryl or halogen groups or Rx and Ry, together with the carbon atom to which they are attached, represent a five- to eight-membered cycloalkylidene group which may bear one or more oxygen, sulphur or nitrogen atoms as hetero atoms, or an adamantylidene or cholestanylidene group, substituted or not substituted by one or more lower alkyl groups, Rw is a lower alkylidene group or Rx and Rw, together with the carbon atom to which they are attatched, represent a five- to eight-membered cycloalkenyl group which may bear one or more oxygen, sulphur or nitrogen atoms as hetero atoms, substituted or not substituted by one or more lower alkyl groups, and Rz is an aryl group substituted or not substituted by one or more lower alkyl groups, characterized in that a compound having the general formula:

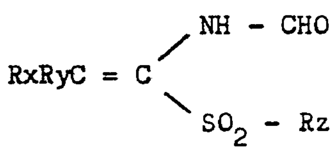 (III)

wherein Rx, Ry and Rz are as indicated above, is reacted with a dehydrating agent.

2. Process according to claim 1, characterized in that the dehydrating agent is phosphorusoxychloride, preferably in the presence of an acid-binding agent.

3. Process according to claim 2, characterized in that the reaction is carried out at lower temperatures, e.g. between -50 and +25°C, preferably between -30 and -5°C.

4. Process according to claim 2 or 3, characterized in that an inert solvent is used, e.g. di-, tri or tetrachloromethane, ethyl acetate, dioxane, benzene, toluene, xylene, o-dichlorobenzene, acetone, 1,2-dimethoxyethane, bis-(2-methoxyethyl)-ether, dimethylformamide,

or 1,2-dichloromethane or mixtures thereof.

5. Process according to anyone of claims 1 to 4, characterized in that the preparation is combined with the preparation of the starting formamide from a compound having the formula:

$$Rz - SO_2 - CH_2 - N = C \qquad (IV)$$

wherein Rz is as indicated above, with a compound having the formula Rx - CO - Ry, wherein Rx and Ry are as indicated above, to a "one pot process".

6. Process for the preparation of formamides having the general formula:

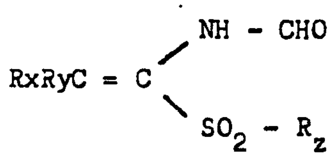

(V)

wherein Rx and Ry are the same or different and each is a hydrogen atom or an alkyl group having 1 to 10 carbon atoms or an aryl group, substituted or not substituted by one or more nitro, halogen, lower alkyl, cycloalkyl, lower alkoxy or aryl groups, or a five- or six-membered heterocyclic group bearing one or more oxygen, sulphur or nitrogen atoms as hetero atoms, substituted or not substituted by one or more lower alkyl, aryl or halogen groups, with the provision that, if Ry is a hydrogen atom, Rx is not a phenyl or t-butyl group, or Rx and Ry, together with the carbon atom to which they are attached, represent a five- to eight-membered cycloalkylidene group which may bear one or more oxygen, sulphur or nitrogen atoms as hetero atoms, with the exception of a cyclohexylidene group, or represent an adamantylidene or cholestanylidene group, substituted or not substituted by one or more lower alkyl groups, and Rz is an aryl group substituted or not substituted by one or more lower alkyl groups, characterized in that the corresponding sulphomethylisocyanide is reacted with the corresponding aldehyde or ketone in the presence of a strong alkaline agent.

7. Process according to claim 6, characterized in that the strong alkaline agent is an alkali metal t-butylate, alkali metal ethanolate, alkali metal hydride, a butylated alkali metal, preferably potassium t-butylate.

8. Process according to claim 7, characterized in that the alkali metal is lithium, sodium or potassium.

9. Process according to any one of claims 6 to 8, characterized in that the reaction is carried out at lower temperatures, e.g. between -60 and +45°C, preferably between -60 and -30°C.

10. Process according to any one of claims 6 to 9, characterized in that the reaction is carried out in an inert gas atmosphere, e.g. a nitrogen gas atmosphere.

11. Process according to any one of claims 6 to 10, characterized in that the reaction is carried out in a polar organic solvent such as tetrahydrofuran, dimethylformamide, 1,2-dimethoxyethane, dimethylsulphoxide, hexamethylphosphorustriamide, dioxane, toluene or mixtures thereof.

European Patent Office

# EUROPEAN SEARCH REPORT

Application number

EP 79 20 0404

| DOCUMENTS CONSIDERED TO BE RELEVANT | | | CLASSIFICATION OF THE APPLICATION (Int. Cl. ³) |
|---|---|---|---|
| Category | Citation of document with indication, where appropriate, of relevant passages | Relevant to claim | |
| A | US - A - 3 159 666 (HEININGER et al.)<br>* Claim 1; column 7, lines 10-35 *<br>-- | 1 | C 07 C 147/08<br>C 07 D 307/52<br>A 61 K 31/275<br>31/34<br>A 01 N 47/40 |
| A | JUSTIS LIEBIGS ANNALEN DER CHEMIE, Band 766, 1972, Verlag Chemie Weinheim, pages 130-141<br>U. SCHOELLKOPF et al.: "Synthesen mit alpha-metallierten Isocyaniden, XVII"<br>* Pages 140-141; part: Arylsulfonyl-methylisocyanide 4 *<br>-- | 1 | TECHNICAL FIELDS SEARCHED (Int.Cl. ³)<br>C 07 C 119/02<br>147/08<br>A 01 N 1/00 |
| A | CH - A - 437 903 (BAYER)<br>* Column 1, line 11 - column 2, line 23 *<br>---- | 1 | |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | The present search report has been drawn up for all claims | | &: member of the same patent family, corresponding document |

| Place of search | Date of completion of the search | Examiner |
|---|---|---|
| The Hague | 24-10-1979 | DE ROY |